(19) Europäisches Patentamt
European Patent Office
Office européen des brevets

(11) Numéro de publication : **0 043 752**
**B1**

(12) # FASCICULE DE BREVET EUROPÉEN

(45) Date de publication du fascicule du brevet : **13.06.84**

(21) Numéro de dépôt : **81401010.4**

(22) Date de dépôt : **23.06.81**

(51) Int. Cl.³ : **C 07 D491/04**, C 07 D491/20, A 61 K 31/40// C07D493/10, C07D311/22, C07D311/58, C07D311/64 ,(C07D491/04, 311/00, 209/00),(C07D491/20, 317/00, 311/00, 209/00),(C07D491/20, 319/00, 311/00, 209/00)

(54) **Dérivé de pyranno-indole et son procédé de préparation.**

(30) Priorité : **26.06.80 FR 8014246**

(43) Date de publication de la demande : **13.01.82 Bulletin 82/02**

(45) Mention de la délivrance du brevet : **13.06.84 Bulletin 84/24**

(84) Etats contractants désignés : **AT BE CH DE FR GB IT LI LU NL SE**

(56) Documents cités :
**FR-A- 2 267 777**
**Le dossier contient des informations techniques présentées postérieurement au dépôt de la demande et ne figurant pas dans le présent fascicule.**

(73) Titulaire : **SOCIETE DE RECHERCHES INDUSTRIELLES S.O.R.I. Société anonyme dite:**
**3, rue de Citeaux**
**F-75012 Paris (FR)**

(72) Inventeur : **Picart, François**
**38, rue Devosge**
**F-21000 Dijon (FR)**

(74) Mandataire : **Richebourg, Michel François et al**
**Cabinet Beau de Loménie 55, rue d'Amsterdam**
**F-75008 Paris (FR)**

**EP 0 043 752 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne en tant que produit industriel nouveau un dérivé de pyranno-indole. Elle concerne également son procédé de préparation et son application en thérapeutique, notamment en tant qu'agents anti-ulcéreux, anti-inflammatoires et antalgiques.

On connaît de FR-A-2 267 777 des dérivés de pyrrolo [3,2-f] chromanne présentés en tant qu'agents antiulcéreux, analgésiques et antiinflammatoires. On vient de trouver que des dérivés structurellement différents du type pyranno-indole sont utiles en thérapeutique.

Le dérivé selon l'invention est choisi parmi l'ensemble constitué par :

(i) les pyranno [2,3-g] indoles de formule générale

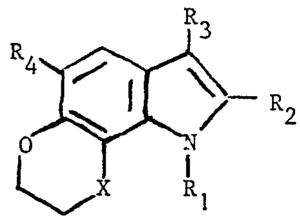

$I_0$

dans laquelle :

X représente un groupe $>CH_2$, $>CHOH$, $>CO$,

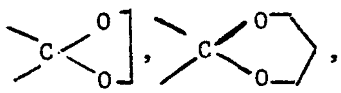

$>C=NR$ (où R est un groupe OH, alkoxy en $C_1$-$C_4$) ou $>CH-NR'R''$ (où R' et R'', identiques ou différents, représentent chacun H ou un alkyle en $C_1$-$C_4$) ;

$R_1$ représente l'atome dl'hydrogène, un groupe alkyle en $C_1$-$C_4$ ou un groupe aminoalkylène $-(CH_2)_n-NR'R''$, dans lequel n est un nombre entier compris entre 1 et 4 et

R' et R'' sont définis comme ci-dessus ;

$R_2$ représente l'atome d'hydrogène ou un groupe COY (où Y est OH ou alkoxy en $C_1$-$C_4$)

$R_3$ représente l'atome d'hydrogène un groupe COH, $(CH_2)_n$ NR'R'' ou CO—CONR'R'' (où n, R' et R'' sont définis comme ci-dessus) ; et

$R_4$ représente l'atome d'hydrogène, un atome d'halogène un groupe d'alkyle en $C_1$-$C_4$ ou alkoxy en $C_1$-$C_4$ ;

(ii) les pyranno [3,2-f] indoles de formule générale

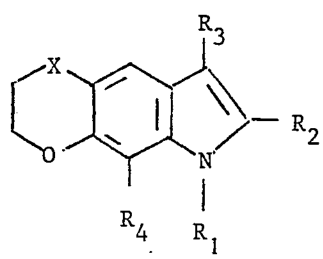

$I'_0$

où X, $R_1$, $R_2$, $R_3$ et $R_4$ sont définis comme ci-dessus ; et

(iii) leurs sels d'addition d'acide.

Par groupe alkyle en $C_1$-$C_4$, on entend ici un reste hydro-carboné à chaîne linéaire ou ramifiée renfermant de 1 à 4 atomes de carbone. Dans le groupe alkoxy en $C_1$-$C_4$, le reste alkyle est un reste hydrocarboné en $C_1$-$C_4$ à chaîne linéaire ou ramifiée. Parmi les groupes alkyle en $C_1$-$C_4$ et alkoxy en $C_1$-$C_4$ qui conviennent on peut notamment citer $CH_3$, $CH_2CH_3$, $CH_2CH_2CH_3$, $CH(CH_3)_2$, $C(CH_3)_3$, $OCH_3$, $OCH_2CH_3$, $OCH(CH_3)_2$ et $OC(CH_3)_3$.

Par atome d'halogène, on entend ici un atome de fluor, de chlore, de brome ou d'iode, les atomes d'halogène préférés étant le brome et le chlore.

De façon non limitative, on a consigné dans les tableaux 1 et 11 ci-après un certain nombre de dérivés de pyranno-indole selon l'invention.

Le procédé de préparation d'un composé pyranno-indole selon l'invention est illustré par les mécanismes réactionnels du diagramme 1 ci-après. Ce procédé est caractérisé en ce que :

a) on prépare un pyranno-indole-carboxylate de formule IX comme suit

(i) on fait réagir l'aldéhyde de formule VII (où X et R sont définis comme ci-dessus) avec l'azidoacétate d'éthyle en présence d'un alcool inférieur en $C_1$-$C_2$ (notamment $C_2H_5OH$ et de préférence $CH_3OH$) et d'un métal alcalin choisi parmi Na et K à une température de $-5\,°C$ à $+5\,°C$ (de préférence à $0\,°C$) pendant au moins 3 heures pour obtenir un azide de formule VIII, et

(ii) on cyclise ledit azide VIII en présence d'un solvant inerte choisi parmi le benzène, le toluène et le xylène, à la température de reflux du milieu réactionnel pendant au moins 1 heure, pour obtenir ledit pyranno-indole-carboxylate IX qui est un dérivé de formule $I_0$ où $R_1 = R_3 = H$ et $R_2$ qui représente un reste carboxylate est de préférence $COOCH_3$, puis

b) si nécessaire, transforme ledit dérivé pyranno-indole-carboxylate en acide, esters et amides correspondants notamment par saponification, transestérification et respectivement transamidification.

Le meilleur mode de préparation de l'ensemble des composés de formules $I_0$ et $I_0'$ (qui est également illustré par le diagramme I ci-après) consiste à

c) saponifier l'ester IX pour obtenir l'acide correspondant X (qui est un dérivé de formule $I_0$ où $R_1 = R_3 = H$ et $R_2 = COOH$),

d) décarboxyler ledit acide X au moyen d'un catalyseur (de préférence le chromite de cuivre préparé à partir de CuO et $Cr_2O_3$) en présence de solvant inerte (de préférence la quinoléine à 180-200 °C sous 1 atmosphère) pour obtenir (i) un mélange des composés XI et XI' quand $R_4 = H$ que l'on sépare notamment par chromatographie sur silice et (ii) uniquement le composé XI lorsque $R_4$ est différent de H, puis,

e) si nécessaire, introduire au moins un des groupes $R_1$ et $R_3$ différents de H pour obtenir, à partir de XI et XI', respectivement les composés XII et XII' ; et, le cas échéant introduire le groupe $R_4$ différent de H pour obtenir le composé XII', à partir du composé XI'.

Bien entendu le groupe acide $R_2 = COOH$ peut être estérifié, amidifié et décarboxylé.

Les composés VII, qui interviennent comme matières premières dans le procédé de synthèse des pyranno-indoles de formules $I_0$ et $I_0'$ peuvent être préparés selon une méthode connue en soi, par application de mécanismes réactionnels classiques.

Selon l'invention on préconise plusieurs routes pour leur synthèse, en fonction de la nature du groupe X. Ces routes sont illustrées par les diagrammes 2 et 3 ci-après.

La route A schématisée par le diagramme 2 vise l'obtention d'un dérivé VII particulier quand le groupe X est $CH_2$. Dans une première variante (quand X est $CH_2$ et $R_4$ est H), on soumet le 6-bromo-chromanne de formule Ia à une réaction avec CuCN en présence de diméthylamine (DMA) pour donner le 6-cyano-chromanne correspondant, qui est ensuite soumis à une réaction de réduction, notamment en présence de Ni et de $NaH_2PO_2$ pour donner le dérivé 6-chromannyl-carbaldéhyde de formule IIa. Dans une deuxième variante (quand X est $CH_2$ et $R_4$ est différent de H), on soumet un 6-bromo-chromanne substitué en position 8 de formule Ib à une réaction avec un organolithien (le butyllithium de préférence) au sein du diméthylformamide, pour obtenir un 5-chromannyl-carbaldéhyde substitué en 8 de formule IIb.

La route B, qui est schématisée par le diagramme 3, vise l'obtention d'un dérivé VII particulier (dans lequel X est $>CO$,

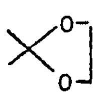 ou 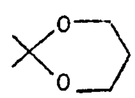 ,

$R_4$ étant quelconque). Cette route consiste à faire réagir une 5-bromo-chromannone de formule III au moyen de $HOCH_2CH_2OH$ ou de $HOCH_2CH_2CH_2OH$ dans un solvant inerte (de préférence un solvant aromatique tel que le benzène, le toluène et le xylène) en présence d'acide p-toluène-sulfonique (ATPS) pour obtenir une dérivé bromé de IV où X est

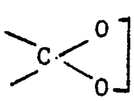 ou 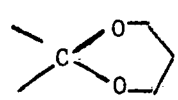 ,

à soumettre le produit IV ainsi obtenu à une réaction avec un organolithien (de préférence le butyllithium) au sein du diméthylformamide pour remplacer le groupe bromo en groupe CHO et obtenir un dérivé de formule V où X est

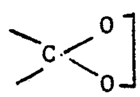 ou 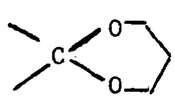 ,

ce dérivé acétal étant, le cas échéant, transformé en dérivé 6-formyl-chromannone de formule VI par hydrolyse acide.

Les composés intermédiaires IIb, IV, V et VI, qui interviennent dans la synthèse des dérivés de pyranno-indole selon l'invention, sont nouveaux.

Ainsi parmi les composés de formules $I_0$ et $I_0'$ selon l'invention sont notamment inclus les composés où X est $>CH_2$, $>CO$,

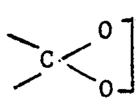 ,

$\gtrdot C = NOH$, CHOH ou CHNH$_2$ ; $R_2$ est H, COOH, CO-alkoxy en $C_1$-$C_4$ ; $R_3$ est H, CHO, $CH_2N(CH_3)_2$ ou CO-CON$(CH_3)_2$ ; $R_4$ est H, Cl, Br, alkyle en $C_1$-$C_4$ ou alkoxy en $C_1$-$C_4$ ; et $R_1$ est H, alkyle en $C_1$-$C_4$ ou $(CH_2)_n$ NR'R" (où n, R' et R" sont définis comme ci-dessus), à savoir :

— les composés de formule $I_0$ où X représente $\gtrdot CH_2$, $R_2$ représente H, COOH ou $COOCH_3$, $R_3$ représente H, CHO, CO-CON$(CH_3)_2$ ou $CH_2N(CH_3)_2$, $R_4$ représente H, Cl ou $OCH_3$, et $R_1$ représente H, $CH_3$ ou un groupe $(CH_2)_n$NR'R" (où n, R' et R" sont définis comme indiqué ci-dessus) ;

— les composés de formule $I_0$ où X représente $\gtrdot CO$, $R_2$ représente H, COOH ou $COOCH_3$, $R_3$ représente H, CHO ou $CH_2N(CH_3)_2$, $R_4$ représente H, Cl ou $OCH_3$ et $R_1$ représente l'atome d'hydrogène ;

— les composés de formule $I_0$ où X représente

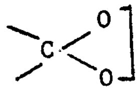,

$R_2$ représente $COOCH_3$, $R_4$ représente H, Cl ou $OCH_3$, et $R_1$ et $R_3$ représentent chacun l'atome d'hydrogène ;

— les composés de formule $I_0'$ où X est $CH_2$, $R_3$ est H ou CHO, $R_1$ et $R_2$ représentent chacun l'atome d'hydrogène , et $R_4$ représente l'atome d'hydrogène ou de chlore ;

— les composés de formule I ou X représente $\gtrdot C = NR$ (où R est un groupe OH ou alkoxy en $C_1$-$C_4$), $R_2$ et $R_3$ représentent chacun l'atome d'hydrogène, $R_1$ représente l'atome d'hydrogène ou un groupe alkyle en $C_1$-$C_4$ et $R_4$ représente H, Cl ou $OCH_3$ ;

— les composés de formule $I_0$ où X représente $\gtrdot CH$—$NH_2$, $R_2$ et $R_3$ représentent chacun l'atome d'hydrogène, $R_1$ représente l'atome d'hydrogène ou un groupe alkyle en $C_1$-$C_4$ et $R_4$ représente H, Cl ou $OCH_3$.

D'autres avantages et caractéristiques de l'invention seront mieux compris à la lecture qui va suivre d'exemples de préparation nullement limitatifs, mais donnés à titre d'illustration. On trouvera ci-après les « préparations I-XI » relatives à l'obtention des matières premières puis les exemples 1-31 consignés dans les tableaux I et II ci-après.

## Préparation I

6-Bromo-8-chloro-chromanne, ($Ib_1$)

A l'amalgame de zinc — préparé à partir de 1 280 g (19,7 atomes-grammes) de zinc, 128 g ($5,4 \times 10^{-1}$ M) de chlorure mercureux, 64 $cm^3$ d'acide chlorhydrique concentré et 1 900 $cm^3$ d'eau — on ajoute dans l'ordre 1 000 $cm^3$ d'eau, 1 500 $cm^3$ d'acide chlorhydrique concentré, 3 500 $cm^3$ de toluène et 640 g (2,45 M) de 6-bromo-8-chloro-4-chromannone ($III_2$). Le mélange est porté à reflux durant 1 h et après refroidissement et décantation il est extrait à l'éther et lavé à l'eau jusqu'à neutralité. On isole ainsi 305 g (rendement = 50 %) de produit attendu. $Eb_{0,01}$ = 128 °C.

Analyse $C_9H_8OBrCl$
Calculé % : C 43,68 H 3,23 Br 32,29
Trouvé % : C 43,5 H 3,2 Br 32,0

## Préparation II

6-Bromo-8-méthoxy-chromanne, ($Ib_2$)

Selon le procédé de la préparation I, au départ de l'amalgame de zinc préparé à partir de 915 g (14 atomes-grammes) de zinc, 91 g ($3,8 \times 10^{-1}$ M) de chlorure mercureux dans 46 $cm^3$ d'acide chlorhydrique concentré et 1 350 $cm^3$ d'eau, après ajout de 700 $cm^3$ d'eau, 1 100 $cm^3$ d'acide chlorhydrique concentré, 2 500 $cm^3$ de toluène et 450 g (1,8 M) de 6-bromo-8-méthoxy-4-chromannone ($III_1$), on obtient 200 g (Rendement = 47 %) de produit attendu. F = 67 °C.

Analyse $C_{10}H_{11}O_2Br$
Calculé % : C 49,42 H 4,52 O 13,16 Br 32,88
Trouvé % : C 49,4 H 4,5 O 13,5 Br 32,6

## Préparation III

8-Chloro-chromanne-6-carbaldéhyde, ($IIb_1$)

72 g ($2,9 \times 10^{-1}$ M) du dihalogénochromanne $Ib_1$ dissous dans 250 $cm^3$ d'éther anhydre sont ajoutés lentement et sous agitation dans $2,9 \times 10^{-1}$ M de n-butyllithium dans 320 $cm^3$ d'éther anhydre. La température est maintenue à − 70 °C durant 6 h puis on additionne goutte à goutte 31 g ($3,8 \times 10^{-1}$ M) de N,N-diméthylformamide dissous dans 150 $cm^3$ d'éther anhydre. Le mélange réactionnel est encore

maintenu sous agitation à la même température durant 12 h puis — après retour à la température ambiante — il est versé sur de l'eau glacée. Après acidification avec l'acide chlorhydrique 5N, extraction à l'éther et évaporation, on isole 54 g (Rendement = 95 %) de 8-chloro-chromanne-6-carbaldéhyde. F = 51 °C.

Analyse $C_{10}H_9O_2Cl$
Calculé % : C 61,11 H 4,57 O 16,27 Cl 18,03
Trouvé % : C 61,2 H 4,6 O 16,3 Cl 18,0

Préparation IV

8-Méthoxy-chromanne-6-carbaldéhyde, ($IIb_2$)

On procède selon la méthode décrite dans la préparation III, mais en maintenant la température à − 50 °C. Au départ de 186 g ($7,7 \times 10^{-1}$ M) de bromo-méthoxy-chromanne $Ib_2$ dans 500 $cm^3$ d'éther anhydre et de $7,7 \times 10^{-1}$ M de n-butyllithium dans 650 $cm^3$ d'éther anhydre on isole, après ajout de 78 g (1,1 M) de N,N-diméthylformamide dans 200 $cm^3$ d'éther anhydre et recristallisation dans l'éther isopropylique, 75 g (Rendement = 51 %) de composé attendu. F = 81 °C.

Analyse $C_{11}H_{12}O_3$
Calculé % : C 68,77 H 6,24 O 24,97
Trouvé % : C 69,0 H 6,3 O 25,2

Préparation V

6-Bromo-8-méthoxy-4-chromannone, ($III_1$)

On ajoute — à 0 °C — 34 g ($3,5 \times 10^{-1}$ M) d'acide phosphorique dans un mélange contenant 475 $cm^3$ de benzène anhydre et 56 g ($4 \times 10^{-1}$ M) d'anhydride phosphorique. Après avoir porté 4 h à ébullition on additionne — lentement — 83 g ($3 \times 10^{-1}$ M) d'acide 3-(4-bromo-2-méthoxy-1-phénoxy)-propionique. Le mélange est maintenu à ébullition durant 12 h, puis après refroidissement versé sur l'eau glacée, traité par l'acide chlorhydrique 5 N et extrait à l'éther. Après traitement habituel et recristallisation du solide obtenu dans l'éthanol, on isole 67 g (Rendement : 86 %) de composé attendu. F = 137 °C.

Analyse $C_{10}H_9O_3Br$
Calculé % : C 46,73 H 3,50 O 18,66 Br 31,09
Trouvé % : C 46,7 H 3,5 O 18,7 Br 31,3

Préparation VI

6-Bromo-8-chloro-4-chromannone, ($III_2$)

On porte durant 2 h à 70-80 °C un mélange de 279 g (1 M) d'acide 3-(4-bromo-2-chloro-1-phénoxy)-propionique et 127 g (1,3 M) d'acide sulfurique concentré. Le mélange réactionnel est ensuite versé sur l'eau glacée, extrait à l'éther, lavé avec une solution diluée d'ammoniaque puis à l'eau. On obtient finalement 183 g (Rendement = 70 %) de produit attendu. F = 109 °C.

Analyse $C_9H_6O_2BrCl$
Calculé % : C 41,34 H 2,29 O 12,23
Trouvé % : C 41,3 H 2,4 O 12,1

Préparation VII

Dioxolanne de la 6-bromo-8-méthoxy-4-chromannone, ($IV_1$)

10 g ($3,9 \times 10^{-2}$ M) de 4-chromannone, $III_1$, 24 g ($3,9 \times 10^{-1}$ M) d'éthylèneglycol et 0,3 g ($1,7 \times 10^{-3}$ M) d'acide paratoluènesulfonique dans 200 $cm^3$ de toluène anhydre sont portés à ébullition durant 7 h dans un appareil DEAN et STARK. Le mélange réactionnel est ensuite versé sur l'eau glacée, extrait à l'éther et le solide obtenu est recristallisé dans un mélange alcool éthylique-éther. On obtient 9,6 g (Rendement = 82 %) de produit attendu. F = 104 °C.

Analyse $C_{12}H_{13}O_4Br$
Calculé % : C 47,88 H 4,31 O 21,25 Br 26,54
Trouvé % : C 47,8 H 4,5 O 21,3 Br 26,4

Préparation VIII

Dioxolanne de la 6-bromo-8-chloro-4-chromannone, ($IV_2$)

De même, 30 g ($1,1 \times 10^{-1}$ M) de 4-chromannone ($III_2$), 71 g (1,1 M) d'éthylèneglycol et 0,2 g ($1,1 \times 10^{-3}$ M) d'acide paratoluènesulfonique dans 200 $cm^3$ de toluène conduisent, après recristallisation dans un mélange benzène-éther isopropylique, à 34 g (Rendement = 97 %) de produit attendu. F = 94 °C.

Analyse $C_{11}H_{10}O_3BrCl$
Calculé % : C 43,25 H 3,27 O 15,70
Trouvé % : C 43,2 H 3,2 O 15,6

Préparation IX

4-Dioxolanno-8-méthoxy-chromanne-6-carbaldéhyde, ($V_1$)

177 g ($5,9 \times 10^{-1}$ M) de dioxolanne $IV_1$ dissous dans 500 $cm^3$ d'éther anhydre sont ajoutés lentement à − 70 °C à $5,9 \times 10^{-1}$ M de n-butyllithium dans 570 $cm^3$ d'éther anhydre. Après addition, on maintient le milieu durant 6 h à − 70 °C et ajoute goutte à goutte 56 g ($7,7 \times 10^{-1}$ M) de N,N-diméthylformamide dissous dans 300 $cm^3$ d'éther anhydre. Le mélange réactionnel est maintenu durant 12 h à − 70 °C puis, après retour à la température ambiante, versé sur l'eau glacée. Après extraction au chloroforme, recristallisation du solide obtenu dans l'éthanol, on isole 133 g (Rendement : 90 %) de produit attendu. F = 167 °C.

Analyse $C_{13}H_{14}O_5$
Calculé % : C 62,43 H 5,59 O 31,96
Trouvé % : C 61,2 H 5,6 O 31,2

Préparation X

8-Chloro-4-dioxolanno-chromanne-6-carbaldéhyde, ($V_2$)

En opérant comme indiqué dans la préparation IX à partir de 45 g ($1,5 \times 10^{-1}$ M) de dioxolanne $IV_2$, on obtient après recristallisation dans le benzène 31 g (rendement : 84 %) de produit attendu. F = 133 °C.

Analyse $C_{12}H_{11}O_4Cl$
Calculé % : C 56,62 H 4,32 O 25,12
Trouvé % : C 55,3 H 4,5 O 24,6

Préparation XI

8-Méthoxy-4-oxo-chromanne-6-carbaldéhyde, ($VI_1$)

A 34 g ($1,4 \times 10^{-1}$ M) du dioxolanne $V_1$ dans 700 $cm^3$ d'alcool éthylique on ajoute — lentement et à température ambiante — 90 $cm^3$ d'acide chlorhydrique concentré. Le mélange est maintenu sous agitation durant 1 h, puis versé sur l'eau glacée et extrait à l'éther. Après recristallisation du solide obtenu dans l'alcool éthylique on obtient 26 g (Rendement = 91 %) de produit attendu. F = 139 °C.

Analyse $C_{11}H_{10}O_4$
Calculé % : C 64,10 H 4,85 O 31,03
Trouvé % : C 64,5 H 4,9 O 30,1

Exemple 1

7,8,9-Trihydro-pyranno [2,3-g] indole-2-carboxylate de méthyle

A une solution méthanolique de méthylate de sodium [1,4 g de Na ($6 \times 10^{-2}$ atomes-grammes) dans 30 $cm^3$ de méthanol anhydre], on ajoute, à 0 °C, 4,5 g ($2,8 \times 10^{-2}$ M) de 6-formyl-chromanne, dissous dans 10 $cm^3$ de méthanol anhydre. On verse alors, goutte à goutte, à 0 °C et sous agitation 7,2 g ($5,6 \times 10^{-2}$ M) d'azidoacétate d'éthyle dans 10 $cm^3$ de méthanol anhydre. Après avoir maintenu la température durant 6 h à 0 °C on verse le mélange réactionnel sur l'eau glacée et retient par filtration l'azide.

Dans 30 $cm^3$ de xylène porté à ébullition, on ajoute sous agitation l'azide dissous dans 150 $cm^3$ de xylène. L'addition terminée, agitation et reflux sont maintenus durant 5 h. Après élimination du xylène, le

résidu solide obtenu est recristallisé dans un mélange éther isopropylique-benzène et on obtient 2,7 g. (Rendement = 42 %) de produit attendu. F = 163 °C.

Analyse $C_{13}H_{13}O_3N$
Calculé % : C 67,55 H 5,62 O 20,75 N 6,05
Trouvé % : C 67,6 H 5,7 O 20,7 N 6,0

Exemple 2

5-Chloro-7,8,9-trihydro-pyranno [2,3-g] indole-2-carboxylate de méthyle

Selon le procédé décrit à l'exemple 1 et au départ de 16 g ($6,9 \times 10^{-1}$ atomes-grammes) de Na dissous dans 600 $cm^3$ de méthanol anhydre, 59 g ($3 \times 10^{-1}$ M) du chloroformyl-chromanne $IIb_1$ dans 250 $cm^3$ de méthanol anhydre et 77 g ($6 \times 10^{-1}$ M) d'azidoacétate d'éthyle dans 100 $cm^3$ de méthanol anhydre, on isole l'azide qui est dissous dans 500 $cm^3$ de xylène et ajouté à 500 $cm^3$ de xylène portés à ébullition. Après élimination du xylène et recristallisation dans l'alcool éthylique, on isole 23 g (Rendement = 29 %) de composé attendu. F = 238 °C.

Analyse $C_{13}H_{12}O_3NCl$
Calculé % : C 58,79 O 18,06 N 5,27 Cl 13,34
Trouvé % : C 58,7 O 18,3 N 5,3 Cl 13,4

Exemple 3

5-Méthoxy-7,8,9-trihydro-pyranno [2,3-g] indole-2-carboxylate de méthyle

Selon le procédé décrit à l'exemple 1, et à partir de 19 g ($8 \times 10^{-1}$ atomes-grammes) de Na dissous dans 1 250 $cm^3$ de méthanol anhydre, 73 g ($3,8 \times 10^{-1}$ M) de méthoxyformylchromanne $IIb_2$ dans 50 $cm^3$ de méthanol et 98 g ($7,6 \times 10^{-1}$ M) d'azidoacétate d'éthyle dans 100 $cm^3$ de méthanol, on isole l'azide. Après cyclisation de l'azide dans le xylène, on obtient 38 g (Rendement = 38 %) d'ester attendu. F = 231 °C.

Analyse $C_{14}H_{15}O_4N$
Calculé % : C 64,39 H 5,74 O 24,49 N 5,36
Trouvé % : C 64,3 H 5,8 O 24,3 N 5,2

Exemple 4

Acide 7,8,9-trihydro-pyranno [2,3-g] indole-2-carboxylique

On porte à reflux durant 3 h 30 une solution de 17 g ($7,4 \times 10^{-2}$ M) du produit de l'exemple 1 et 8,3 g ($1,5 \times 10^{-1}$ M) de KOH dans 300 $cm^3$ d'eau. Après retour à la température ambiante, acidification à 0 °C par l'acide chlorhydrique 5N, le précipité obtenu est filtré, lavé à l'eau jusqu'à neutralité et recristallisé dans un mélange éthanol-eau. On isole 13,2 g (Rendement = 83 %) d'acide attendu F = 228 °C.

Analyse $C_{12}H_{11}O_3N$
Calculé % : C 66,38 H 5,06 O 22,09 N 6,44
Trouvé % : C 66,5 H 5,2 O 22,1 N 6,4

Exemple 5

Acide 5-chloro-7,8,9-trihydro-pyranno [2,3-g] indole-2-carboxylique

On opère selon le procédé de l'exemple 4, au départ de 22,6 g ($8,5 \times 10^{-2}$ M) de produit de l'exemple 4, au départ de 22,6 g ($8,5 \times 10^{-2}$ M) de produit de l'exemple 2, et de 14 g ($2,6 \times 10^{-1}$ M) de potasse dans 300 $cm^3$ d'eau, on obtient 20 g (Rendement = 93 %) d'acide attendu. F = 308 °C.

Analyse $C_{12}H_{10}O_3NCl$
Calculé % : C 57,29 H 3,97 O 19,07 N 5,56 Cl 14,09
Trouvé % : C 57,1 H 4,0 O 19,2 N 5,6 Cl 13,7

Exemple 6

Acide 5-méthoxy-7,8,9-trihydro-pyranno [2,3-g] indole-2-carboxylique

Selon le procédé de l'exemple 4 et à partir de 10 g ($3,8 \times 10^{-2}$ M) du produit de l'exemple 3 dans 150 $cm^3$ de méthanol et de 6,4 g ($1,1 \times 10^{-1}$ M) de potasse dans 15 $cm^3$ d'eau portés à ébullition durant 1 h 30, on isole 9,3 g (Rendement = 98 %) d'acide désiré. F = 268 °C.

Analyse $C_{13}H_{13}O_4N$
Calculé % : C 63,18 H 5,26 O 25,88 N 5,66
Trouvé % : C 63,1 H 5,3 O 26,0 N 5,8

Exemples 7 et 29

7,8,9-Trihydro-pyranno [2,3-g] indole, exemple 7, et 5,6,7-trihydro-pyranno [3,2-f] indole, exemple 29

On chauffe à 190-195 °C (durant 7 h 30) 120 $cm^3$ de quinoléine distillée, 5 g ($2,3 \times 10^{-2}$ M) du produit de l'exemple 4 et 80 mg de chromite de cuivre. Après retour à la température ambiante, on recueille le catalyseur par filtration, dilué avec du chloroforme et élimine la quinoléine par lavage à l'acide chlorhydrique 5 N. La phase chloroformique est ensuite lavée à l'eau jusqu'à neutralité, évaporée et le résidu obtenu est chromatographié sur acide silicique [éluant : chloroforme-hexane (2 : 1) v/v]. On isole après évaporation des fractions d'élution convenables et recristallisation dans l'éther isopropylique 3,3 g (Rendement = 83 %) du pyranno [g] indole désiré ; F = 115 °C et 0,3 g (Rendement = 7 %) du pyranno [f] indole désiré F = 97 °C.

Analyse $C_{11}H_{11}ON$
Calculé % : C 76,32 H 6,35 O 9,23 N 8,08
exemple 7 Trouvé % : C 76,3 H 6,5 O 9,3 N 8,0
exemple 29 Trouvé % : C 75,7 H 6,5 O 9,2 N 8,0

Exemple 8

5-Chloro-7,8,9-trihydro-pyranno [2,3-g] indole

Selon le procédé de l'exemple 7 et au départ de 9 g ($3,6 \times 10^{-2}$ M) du produit de l'exemple 5, et de 0,18 g de chromite de cuivre dans 240 $cm^3$ de quinoléine, après chromatographie sur acide silicique (éluant : chloroforme) et recristallisation dans un mélange éther isopropylique-hexane, on isole 5,5 g (Rendement = 74 %) du pyranno [2,3-g] indole désiré. F = 108 °C.

Analyse $C_{11}H_{10}ONCl$
Calculé % : C 63,65 H 4,81 O 7,70 N 6,74 Cl 17,08
Trouvé % : C 63,8 H 4,9 O 7,9 N 6,8 Cl 17,2

Exemple 9

5-Méthoxy-7,8,9-trihydro-pyranno [2,3-g] indole

Selon le procédé de l'exemple 7, mais avec une durée de décarboxylation réduite (4 h), à partir de 12 g ($4,9 \times 10^{-2}$ M) du produit de l'exemple 6, 0,24 g de chromite de cuivre et 240 $cm^3$ de quinoléine, après chromatographie sur acide silicique (éluant : chloroforme) et recristallisation dans le benzène, on isole 6 g (Rendement = 60 %) de produit attendu. F = 138 °C.

Analyse $C_{12}H_{13}O_2N$
Calculé % : C 70,96 H 6,40 O 15,74 N 6,89
Trouvé % : C 71,0 H 6,4 O 15,8 N 6,8

Exemple 10

7,8,9-Trihydro-pyranno [2,3-g] indole-3-carbaldéhyde

A 0,3 g ($2 \times 10^{-3}$ M) d'oxytrichlorure de phosphore dans 0,12 g ($1,6 \times 10^{-3}$ M) de N,N-diméthylformamide, on verse à 5 °C 10 $cm^3$ de 1,2-dichloroéthane puis 0,27 g ($1,5 \times 10^{-3}$ M) du produit de l'exemple 7 dissous dans 10 $cm^3$ de 1,2-dichloroéthane. Le mélange est maintenu à 5 °C durant 2 h 30 puis porté à reflux pendant 30 min. Après refroidissement on ajoute 1,2 g ($9 \times 10^{-3}$ M) d'acétate de sodium trihydraté dissous dans 10 $cm^3$ d'eau et on porte à ébullition 15 min. Après retour à la température ambiante, neutralisation, lavage à l'eau, on obtient — après recristallisation dans l'éthanol — 0,2 g (Rendement = 64 %) de l'aldéhyde désiré. F = 232 °C.

Analyse $C_{12}H_{11}O_2N$
Calculé % : C 71,66 H 5,46 O 15,90 N 6,96
Trouvé % : C 71,6 H 5,4 O 15,9 N 7,0

Exemple 11

5-Chloro-7,8,9-trihydro-pyranno [2,3-g] indole-3-carbaldéhyde

A partir de 0,3 g ($2 \times 10^{-3}$ M) d'oxytrichlorure de phosphore, 0,12 g ($1,6 \times 10^{-3}$ M) de N,N-diméthylformamide dans 10 cm$^3$ de 1,2-dichloroéthane et 0,3 g ($1,45 \times 10^{-3}$ M) de pyranno [g] indole chloré préparé selon l'exemple 8, on obtient après chromatographie sur acide silicique [éluant : chloroforme-acétone (3 : 1) v/v] et recristallisation dans un mélange benzène-hexane 0,1 g (Rendement = 29 %) de l'aldéhyde désiré. F = 214 °C.

Analyse $C_{12}H_{10}O_2NCl$
Calculé % : C 61,18 H 4,24 O 13,57
Trouvé % : C 59,8 H 4,3 O 13,8

Exemple 12

5-Méthoxy-7,8,9-trihydro-pyranno [2,3-g] indole-3-carbaldéhyde

Selon le procédé de l'exemple 10 et à partir de 0,3 g ($1,47 \times 10^{-3}$ M) de pyranno [g] indole méthoxylé de l'exemple 9, on isole, après chromatographie sur acide silicique [éluant : chloroformeacétone (3 : 1) v/v] et recristallisation dans le benzène, 0,11 g (Rendement = 31 %) de l'aldéhyde attendu, F = 107 °C.

Analyse $C_{13}H_{13}O_3N$
Calculé % : C 67,55 H 5,62 O 20,75 N 6,05
Trouvé % : C 67,1 H 5,6 O 20,8 N 6,0.

Exemple 13

3-(7,8,9-Trihydro-pyranno [2,3-g] indolyl)-N,N-diméthylglyoxamide

A 1 g ($5,7 \times 10^{-3}$ M) de pyranno [g] indole préparé selon l'exemple 7 dans 25 cm$^3$ d'éther anhydre, on verse — sous courant d'azote et à 0 °C — une solution de 1 cm$^3$ de chlorure d'oxalyle dans 5 cm$^3$ d'éther anhydre. On maintient 30 min à 0 °C puis porte 15 min à reflux ; après refroidissement à 0 °C on ajoute rapidement une solution de diméthylamine jusqu'à ce que le milieu réactionnel soit basique. On abandonne 12 h à température ambiante, filtre le précipité apparu et le recristallise dans le méthanol. On isole 1,1 g (Rendement = 70 %) du dérivé désiré. F = 290 °C.

Analyse $C_{15}H_6O_3N_2$
Calculé % : C 66,20 H 5,87 N 10,28
Trouvé % : C 65,3 H 6,0 N 10,0

Exemple 14

3-(7,8,9-Trihydro-pyranno [2,3-g] indolyl)-N,N-diméthylméthylamine

On porte à ébullition durant 3 jours un mélange de 0,46 g ($5,7 \times 10^{-3}$ M) de formaldéhyde à 37 % p/v, 0,65 g ($5,7 \times 10^{-3}$ M) de diméthylamine à 40 % p/v et 1 g ($5,7 \times 10^{-3}$ M) de produit de l'exemple 7 dans 30 cm$^3$ de méthanol. Après évaporation du solvant, dissolution du produit huileux obtenu dans l'acétate d'éthyle, on traite par l'acide chlorhydrique 5 N, lave au chloroforme et rend la phase aqueuse alcaline par ajout de soude à 20 % p/v. On extrait alors par l'éther et isole 0,77 g (Rendement : 58 %) de produit huileux (base libre).

On ajoute 0,8 g ($3,4 \times 10^{-3}$ M) d'acide picrique dissous dans 15 cm$^3$ d'éthanol à température ambiante, agite durant 12 h le mélange réactionnel, filtre le précipité formé, le lave à l'éthanol et obtient ainsi 1,4 g (Rendement = 93 %) de picrate. F = 160 °C.

Analyse $C_{20}H_{21}O_8N_5$
Calculé % : C 52,31 H 4,57 O 27,86 N 15,24
Trouvé % : C 52,3 H 4,5 O 27,9 N 15,2

La préparation des produits des exemples 15 et 16 est donnée dans les modalités opératoires des exemples 17 et respectivement 18.

Exemple 17

5-Méthoxy-9-oxo-7,8-dihydro-pyranno [2,3-g] indole-2-carboxylate de méthyle

A 0,7 g ($3 \times 10^{-2}$ atomes-grammes) de Na dans 30 $cm^3$ de méthanol anhydre, on ajoute 3 g ($1,2 \times 10^{-2}$ M) de dioxolanne $V_1$ et 3,7 g ($2,9 \times 10^{-2}$ M) d'azidoacétate d'éthyle dans 10 $cm^3$ de méthanol anhydre. On maintient 7 jours à 0 °C et extrait à l'éther. L'azide obtenu est ensuite dissous dans 70 $cm^3$ de xylène portés à ébullition durant 3 h et le produit cyclisé ainsi obtenu (exemple 15) est traité après dissolution dans 50 $cm^3$ d'éthanol par 5 $cm^3$ d'acide chlorhydrique concentré. On abandonne durant 18 h et isole après recristallisation dans le benzène 0,52 g (Rendement = 16 %) de l'ester désiré. F = 188 °C.

Analyse $C_{14}H_{13}O_5N$
Calculé % : C 61,12 H 4,72 O 29,06 N 5,08
Trouvé % : C 60,9 H 4,6 O 29,2 N 5,1

Exemple 18

5-Chloro-9-oxo-7,8-dihydro-pyranno [2,3-g] indole-2-carboxylate de méthyle.

A partir de 26 g de l'aldéhyde dioxolanne $V_2$ et selon le procédé décrit pour la préparation du produit précédent, on obtient après cyclisation de l'azide (temps de reflux 8 h) le produit de l'exemple 16 qui, après hydrolyse, donne 15 g (Rendement = 54 %) de l'ester désiré. F = 194 °C. (Recristallisation dans le benzène).

Analyse $C_{13}H_{10}O_4NCl$
Calculé % : C 55,85 H 3,57 O 22,88 N 5,00 Cl 12,68
Trouvé % : C 55,9 H 3,7 O 22,8 N 5,0 Cl 12,8

Exemple 19

Acide 5-méthoxy-9-oxo-7,8-dihydro-pyranno [2,3-g] indole-2-carboxylique

En utilisant la méthode de saponification décrite pour la préparation du produit de l'exemple 4 et à partir de 34 g ($1,2 \times 10^{-1}$ M) du produit de l'exemple 17 et 14 g ($2,5 \times 10^{-1}$ M) de potasse dans 700 $cm^3$ d'eau, on isole 30 g (Rendement = 93 %) d'acide attendu. F = 312 °C.

Analyse $C_{13}H_{11}O_5N$
Calculé % : C 59,80 H 4,21 O 30,62 N 5,36
Trouvé % : C 59,0 H 4,2 O 30,8 N 5,3

Exemple 20

Acide 5-chloro-9-oxo-7,8-dihydro-pyranno [2,3-g] indole-2-carboxylique

Selon le procédé de l'exemple 19 et à partir de 26 g ($9,3 \times 10^{-2}$ M) du produit de l'exemple 8, on obtient 27 g (Rendement = 90 %) d'acide attendu. F = 227-230 °C.

Analyse $C_{12}H_8O_4NCl$
Calculé % : C 54,27 H 3,01 O 24,08 N 5,27
Trouvé % : C 54,2 H 3,6 O 24,7 N 5,0

Exemple 21

5-Méthoxy-9-oxo-7,8-dihydro-pyranno [2,3-g] indole

Selon la méthode de décarboxylation utilisée pour l'obtention du pyrannoindole de l'exemple 7, et à partir de 31 g ($1,2 \times 10^{-1}$ M) du produit de l'exemple 19 et de 0,77 g de chromite de cuivre dans 500 $cm^3$ de quinoléine, on isole, après 13 h de réaction, chromatographie sur acide silicique (éluant : chloroforme) et recristallisaton dans le benzène, 14 g (Rendement = 54 %) de produit attendu. F = 123 °C.

Analyse $C_{12}H_{11}O_3N$
Calculé % : C 66,38 H 5,06 O 22,09 N 6,44
Trouvé % : C 66,4 H 5,1 O 22,2 N 6,3

Exemple 22

5-Méthoxy-9-oxo-7,8-dihydro-pyranno [2,3-g] indole-3-carbaldéhyde

Selon les modalités opératoires décrites pour la préparation du produit de l'exemple 10 et au départ de 0,4 g (1,8 × $10^{-3}$ M) du produit de l'exemple 21, on obtient, après chromatographie sur acide silicique [éluant : chloroforme-acétone (3 : 1) v/v] puis recristallisation dans un mélange éthanol-benzène-hexane, 0,19 g (Rendement = 42 %) de produit attendu. F = 236 °C.

Analyse $C_{13}H_{11}O_4N$
Calculé % : C 63,70 H 4,48 O 26,09 N 5,71
Trouvé % : C 63,4 H 4,6 O 25,8 N 5,7

Les produits des exemples 23 à 28 donnés dans le tableau I ci-après sont préparés selon les modalités opératoires décrites ci-dessus.

Exemple 30

5,6,7-Trihydro-pyranno [3,2-f] indole-3-carbaldéhyde

Selon le procédé de l'exemple 10 et à partir de 0,2 g (1,3 × $10^{-3}$ M) d'oxytrichlorure de phosphore, 0,09 g (1,2 × $10^{-3}$ M) de N,N-diméthylformamide, 0,2 g (1,1 × $10^{-3}$ M) du produit de l'exemple 29 et 1 g (7 × $10^{-3}$ M) d'acétate de sodium trihydraté, on isole, après recristallisation dans le benzène, 0,075 g (Rendement = 31 %) de produit attendu. F = 195 °C.

Analyse $C_{12}H_{11}O_2N$
Calculé % : C 71,66 H 5,46 O 15,90 N 6,96
Trouvé % : C 71,8 H 5,5 O 15,9 N 7,0

Le produit de l'exemple 31 donné dans le tableau II ci-après est préparé selon les modalités opératoires décrites ci-dessus.

Les composés selon l'invention ont essentiellement des effets anti-ulcéreux, anti-inflammatoires et antalgiques. Ils sont notamment utiles en tant qu'agents anti-ulcéreux, anti-inflammatoires et antalgiques dans le traitement des ulcères, des algies et des inflammations. Certains produits présentent en outre des effets antiagrégants et antithrombotiques utiles dans le traitement des maladies liées aux désordres circulatoires.

On a résumé ci-après les essais pharmacologiques qui ont été entrepris en ce qui concerne notamment la toxicité, les ulcères provoqués par l'aspirine et la sérotonine, et les effets anti-inflammatoires et antalgiques. Les méthodes utilisées ont été rappelées ci-après.

Ulcère à l'aspirine

L'expérimentation est réalisée sur des rats mâles WISTAR de 180 à 200 g.

A t = 0, les rats sont mis à jeun et on pratique une première administration du produit à tester à la dose de 100 mg/kg IP.

A t = 18 heures, on administre per os 2 ml d'une suspension ulcérigène à 192 mg d'aspirine/kg, puis on pratique une deuxième administration du produit à tester à la dose de 100 mg/kg IP.

A t = 22 heures, les animaux sont sacrifiés et on cote les ulcères :

— petits ulcères punctiformes : note 1
— ulcères plus étendus : note 3
— ulcères très étendus ou très profonds : note 9.

Les résultats relatifs à l'inhibition des ulcères provoqués par l'aspirine sont donnés dans le tableau III ci-après.

Ulcère à la sérotonine

La technique utilisée est celle décrite par HASHIZUME Arch. Int. Pharmacodyn. 236, 96-108 (1978).

L'expérimentation est réalisée sur 3 lots de rats mâles Sprague-Dawley. Chaque lot comporte 20 animaux.

Le premier lot constitue les témoins, le deuxième est traité par la Carbénoxolone, le troisième est traité par le produit à tester.

Les animaux sont mis à jeun 24 heures avant expérimentation.

A t = 0, on administre :

— le produit à tester par voie IP au troisième lot.
— 100 mg/kg p.o. de Carbénoxolone au deuxième lot.

A t = + 10 min, on pratique une injection sous-cutanée de sérotonine à 60 mg/kg aux trois lots.

A t = + 250 min, les animaux sont sacrifiés. On prélève et étale les estomacs et on cote les ulcères :

— petit ulcère + = 1
— ulcère moyen ++ = 3
— gros ulcère +++ = 9

Les résultats relatifs à l'inhibition des ulcères provoqués par la sérotonine sont donnés dans le tableau IV ci-après avec les doses des produits testés.

Les techniques relatives aux effets anti-inflammatoires (œdème à la carragénine) et antalgique

(cramping) qui ont été utilisées sont les techniques classiques décrites dans la littérature. Les résultats qui ont été obtenus sont consignés dans le tableau III ci-après.

Le tableau III ci-après a trait à la $DL_{50}$ et à l'inhibition des ulcères à l'aspirine, à l'inhibition de l'œdème à la carragénine et au cramping (les produits à tester étant, dans ces deux derniers cas, administrés par voie IP à une dose égale au dixième de la $DL_{50}$ IP).

On préconise selon l'invention une composition thérapeutique, caractérisée en ce qu'elle renferme, en association avec un excipient physiologiquement acceptable, une dose pharmaceutiquement efficace d'un dérivé pyranno-indole selon l'invention, ou l'un de ses sels d'addition d'acide non toxiques.

En clinique, on a obtenu d'excellents résultats chez l'homme, dans le traitement des ulcères avec les produits des exemples 7, 8, 14, 19, 21 et 23, dans le traitement des algies avec le produit de l'exemple 6, et dans le traitement des œdèmes avec les produits des exemples 4, 5, 9 et 24.

La posologie préconisée pour le traitement des ulcères gastriques ou duodénaux, chez l'homme adulte, est de 50 mg à 1 500 mg par jour, par administration orale. Une telle dose peut être répartie en 2 à 5 prises par jour. De préférence, on administrera par voie orale 100 mg à 1 000 mg d'ingrédient actif en 4 ou 5 prises par jour, pendant au moins 2 à 3 semaines.

(Voir Tableaux pages suivantes)

Tableau I

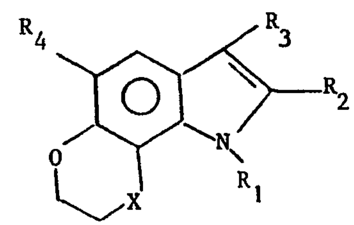

| Exemple | N° code | X | $R_1$ | $R_2$ | $R_3$ | $R_4$ | F. °C |
|---|---|---|---|---|---|---|---|
| 1 | - | $CH_2$ | H | $COOCH_3$ | H | H | 163 |
| 2 | - | $CH_2$ | H | $COOCH_3$ | H | Cl | 238 |
| 3 | - | $CH_2$ | H | $COOCH_3$ | H | $OCH_3$ | 231 |
| 4 | 231 | $CH_2$ | H | COOH | H | H | 228 |
| 5 | 258 | $CH_2$ | H | COOH | H | Cl | 308 |
| 6 | 336 | $CH_2$ | H | COOH | H | $OCH_3$ | 268 |
| 7 | 275 | $CH_2$ | H | H | H | H | 115 |
| 8 | 338 | $CH_2$ | H | H | H | Cl | 108 |
| 9 | 337 | $CH_2$ | H | H | H | $OCH_3$ | 138 |
| 10 | - | $CH_2$ | H | H | CHO | H | 232 |
| 11 | - | $CH_2$ | H | H | CHO | Cl | 214 |
| 12 | - | $CH_2$ | H | H | CHO | $OCH_3$ | 107 |

Tableau I (Suite)

| Exemple | N° code | X | $R_1$ | $R_2$ | $R_3$ | $R_4$ | F.°C |
|---|---|---|---|---|---|---|---|
| 13 | - | $CH_2$ | H | H | 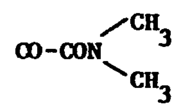 | H | 290 |
| 14 | 333 | $CH_2$ | H | H | 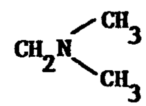 | H | - (a) |
| 15 | - | 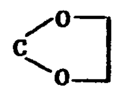 | H | $COOCH_3$ | H | $OCH_3$ | - (b) |
| 16 | - | 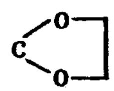 | H | $COOCH_3$ | H | Cl | - (b) |
| 17 | - | CO | H | $COOCH_3$ | H | $OCH_3$ | 188 |
| 18 | - | CO | H | $COOCH_3$ | H | Cl | 194 |
| 19 | 266 | CO | H | COOH | H | $OCH_3$ | 312 |
| 20 | - | CO | H | COOH | H | Cl | 227-230 |
| 21 | 205 | CO | H | H | H | $OCH_3$ | 123 |
| 22 | - | CO | H | H | CHO | $OCH_3$ | 236 |

Note : (a) la base libre est une huile, le picrate correspondant fond à 160°C;
(b) huile.

Tableau I (Fin)

| Exemple | No Code | X | $R_1$ | $R_2$ | $R_3$ | $R_4$ | F °C |
|---|---|---|---|---|---|---|---|
| 23 | 598 | C = NOH | H | H | H | $OCH_3$ | 222 |
| 24 | 599 | CHOH | H | H | H | $OCH_3$ | 147 |
| 25 | - | $CHNH_2$ | H | H | H | $OCH_3$ | (b) |
| 26 | - | CO | H | H | $CH_2N(CH_3)_2$ | $OCH_3$ | 121 |
| 27 | - | $CH_2$ | $(CH_2)_3N(CH_3)_2$ | H | H | Cl | 69 |
| 28 | - | $CH_2$ | $CH_3$ | H | H | Cl | 152 |

Note : (b) Huile.

Tableau II

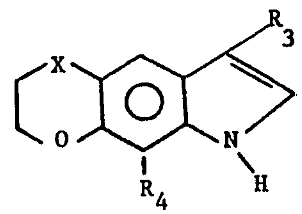

| Exemple | Numéro de code | X | $R_3$ | $R_4$ | F.°C |
|---|---|---|---|---|---|
| 29 | - | $CH_2$ | H | H | 97 |
| 30 | - | $CH_2$ | CHO | H | 195 |
| 31 | - | $CH_2$ | H | Cl | 117 |

Tableau III

| Exemple | N° de code | $DL_{50}$ mg/kg I.P. | Ulcères à l'aspirine % inhibition | Oedème à la carragénine % inhibition | Cramping % inhibition |
|---|---|---|---|---|---|
| 21 | 205 | 600 | 60 | 49 | 37 |
| 4 | 231 | 600 | 11 | 35 | 36 |
| 5 | 258 | 300 | 28 | 30 | 35 |
| 7 | 275 | 750 | 46 | - | 14 |
| 19 | 266 | > 1000 | 49 | - | - |
| 14 | 333 | 180 | 47,5 | 22 | 67 |
| 6 | 336 | > 800 | 23 | - | 66 |
| 9 | 337 | 650 | 51 | 39 | 41 |
| 8 | 338 | 900 | 42,5 | 33 | 26 |
| 23 | 598 | ($DL_0$ > 800) | 40 | 52 | 51 |
| 24 | 599 | 1000 | - | 40 | 60 |

Tableau IV

| Exemple | No Code | Dose mg/kg IP | Ulcères à la serotonine % inhibition |
|---|---|---|---|
| 7 | 275 | 75 | 64 |
| 8 | 338 | 45 | 75 |
| 21 | 205 | 60 | 87 |
| 23 | 598 | 92 | 52 |
| Carbenoxolone | - | 60 | 64 |

Diagramme 1

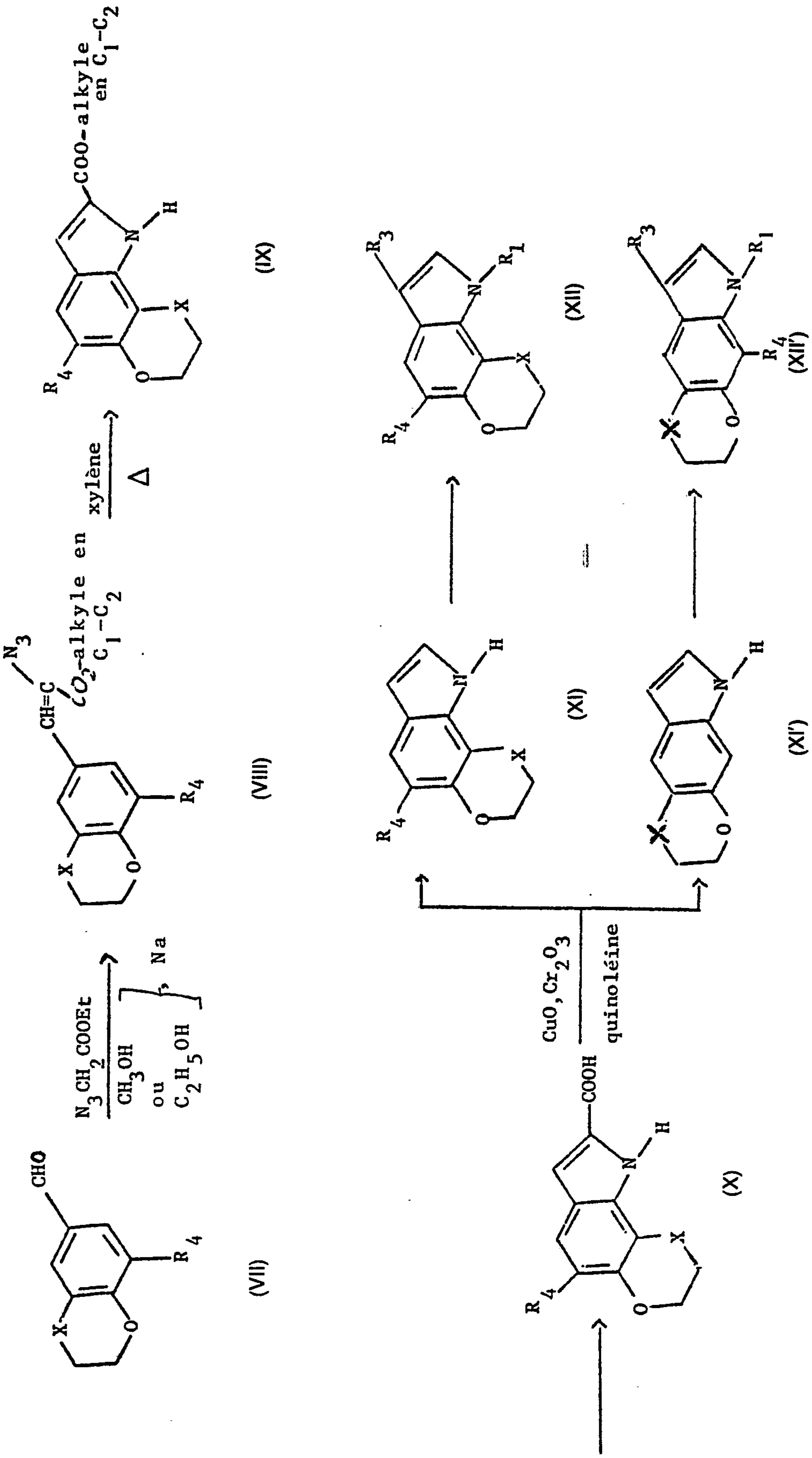


(VII) (VIII) (IX) (X) (XI) (XI') (XII) (XII')

Diagramme 2

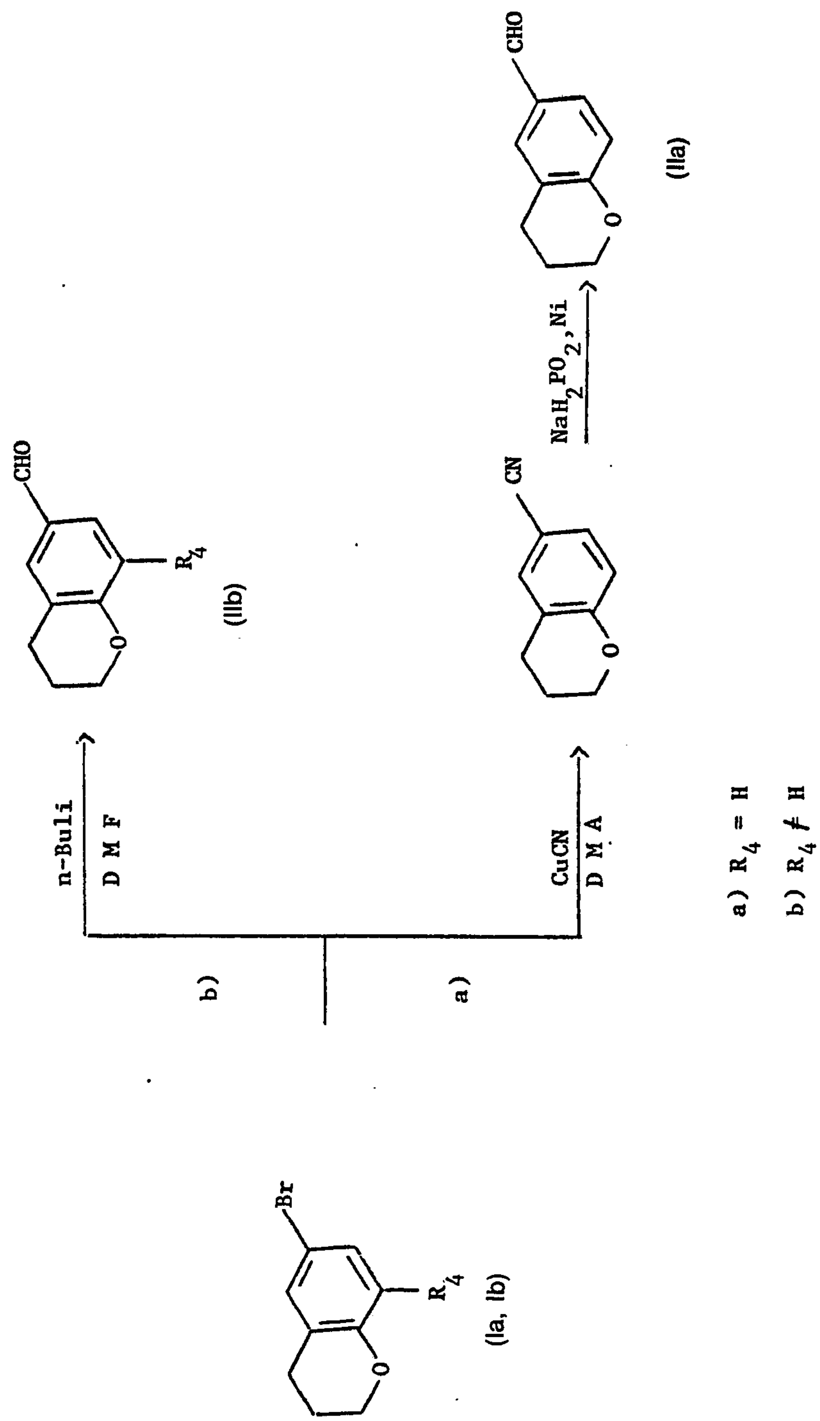


(IIa)

(IIb)

(Ia, Ib)

Diagramme 3

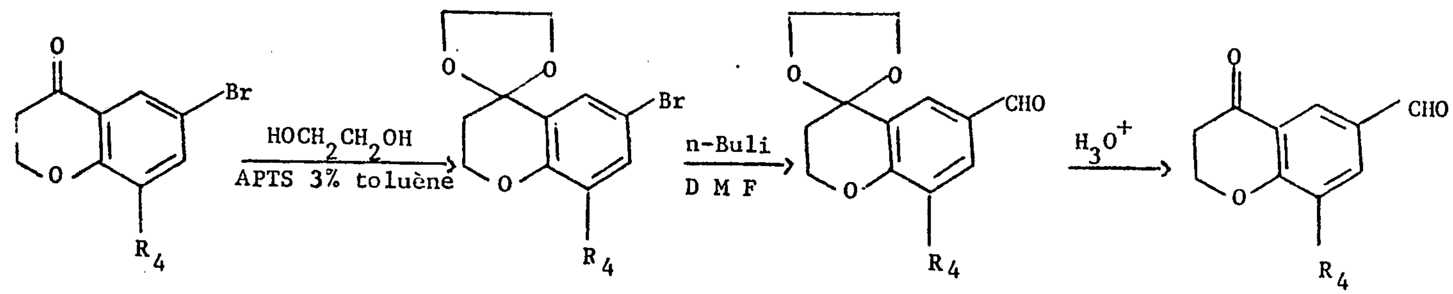


(III) (IV) (V) (VI)

**Revendications** (Pour les Etats contractants : BE, CH, DE, FR, GB, IT, LI, LU, NL, SE)

1. Dérivé appartenant à la famille des pyranno-indoles, caractérisé en ce qu'il est choisi parmi l'ensemble constitué par

(i) les pyranno [2,3-g] indoles de formule générale :

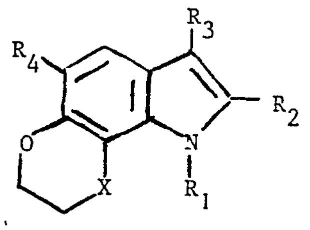

$(I_0)$

dans laquelle

X représente un groupe $>CH_2$, $>CHOH$, $>CO$,

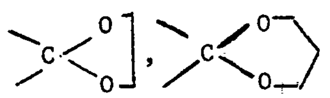

$>C=NR$ (où R est un groupe OH ou alkoxy en $C_1$-$C_4$) ou $>CH—NR'R''$ (où R' et R'', identiques ou différents, représentent chacun H ou un alkyle en $C_1$-$C_4$) ;

$R_1$ représente l'atome d'hydrogène, un groupe alkyle en $C_1$-$C_4$ ou un groupe aminoalkylène $—(CH_2)_n—NR'R''$ dans lequel n est un nombre entier compris entre 1 et 4 et R' et R'' sont définis comme ci-dessus ;

$R_2$ représente l'atome d'hydrogène ou un groupe COY (où Y est OH ou alkoxy en $C_1$-$C_4$) ;

$R_3$ représente l'atome d'hydrogène, un groupe CHO, $(CH_2)_nNR'R''$ ou CO—CONR'R'' (où n, R' et R'' sont définis comme ci-dessus), et

$R_4$ représente l'atome d'hydrogène, un atome d'halogène, un groupe alkyle en $C_1$-$C_4$ ou alkoxy en $C_1$-$C_4$ ;

(ii) les pyranno [3,2-f] indoles de formule générale

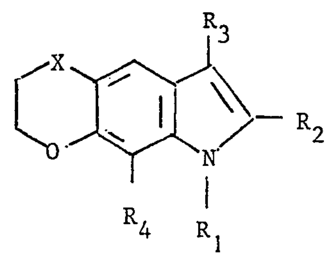

$(I_0')$

où X, $R_1$, $R_2$, $R_3$ et $R_4$ sont définis comme ci-dessus ; et

(iii) leurs sels d'addition d'acide.

2. Dérivé selon la revendication 1, caractérisé en ce que X est $>CH_2$, $>CO$,

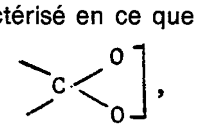

$>C=NOH$, $>CHOH$ ou $>CHNH_2$ ; $R_2$ est H, COOH, CO-alkoxy en $C_1$-$C_4$ ; $R_3$ est H, CHO, $CH_2N(CH_3)_2$ ou $CO—CON(CH_3)_2$ ; $R_4$ est H, Cl, Br, alkyle en $C_1$-$C_4$ ou alkoxy en $C_1$-$C_4$ ; et $R_1$ est H, alkyle en $C_1$-$C_4$ ou $(CH_2)_nNR'R''$ (où n, R' et R'' sont définis comme ci-dessus).

3. Dérivé selon la revendication 1, et répondant à la formule $I_0$, caractérisé en ce que X représente $>CH_2$, $R_2$ représente H, COOH ou $COOCH_3$, $R_3$ représente H, CHO, $CO—CON(CH_3)_2$ ou $CH_2N(CH_3)_2$, $R_4$ représente H, Cl ou $OCH_3$, et $R_1$ représente H, $CH_3$, ou un groupe $(CH_2)_nNR'R''$ (où n, R' et R'' sont définis comme indiqué ci-dessus).

4. Dérivé selon la revendication 1 et répondant à la formule $I_0$, caractérisé en ce que X représente $>CO$, $R_2$ représente H, COOH ou $COOCH_3$, $R_3$ représente H, CHO ou $CH_2N(CH_3)_2$, $R_4$ représente H, Cl ou $OCH_3$, et $R_1$ représente l'atome d'hydrogène.

5. Dérivé selon la revendication 1 et répondant à la formule $I_0$, caractérisé en ce que X représente

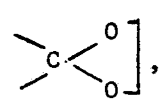

$R_2$ représente $COOCH_3$, $R_4$ représente H, Cl ou $OCH_3$, et $R_1$ et $R_3$ représentent chacun l'atome d'hydrogène.

6. Dérivé selon la revendication 1 et répondant à la formule $I_0'$, caractérisé en ce que X est $CH_2$, $R_3$ est H ou CHO, $R_1$ et $R_2$ représentent chacun l'atome d'hydrogène, et $R_4$ représente l'atome d'hydrogène ou de chlore.

7. Dérivé selon la revendication 3, caractérisé en ce qu'il s'agit de l'acide 5-chloro-7,8,9-trihydropyranno [2,3-g] indole-2-carboxylique.

8. Dérivé selon la revendication 3, caractérisé en ce qu'il s'agit du 5-méthoxy-7,8,9-trihydropyranno [2,3-g] indole.

9. Dérivé selon la revendication 3, caractérisé en ce qu'il s'agit de la 3-(7,8,9-trihydropyranno [2,3-g] indolyl)-N,N-diméthylméthylamine et de ses sels d'addition d'acide.

10. Dérivé selon la revendication 4, caractérisé en ce qu'il s'agit du 5-méthoxy-9-oxo-7,8-dihydropyranno [2,3-g] indole.

11. Dérivé selon la revendication 4, caractérisé en ce qu'il s'agit de l'acide 5-méthoxy-9-oxo-7,8-dihydropyranno [2,3-g] indole-2-carboxylique.

12. Dérivé selon la revendication 6, caractérisé en ce qu'il s'agit du 5,6,7-trihydropyranno [3,2-f] indole-3-carboxaldéhyde.

13. Dérivé selon la revendication 1 et répondant à la formule $I_0$, caractérisé en ce que X représente $>C=NR$ (où R est un groupe OH ou alkoxy en $C_1$-$C_4$), $R_2$ et $R_3$ représentent chacun l'atome d'hydrogène, $R_1$ représente l'atome d'hydrogène ou un groupe alkyle en $C_1$-$C_4$ et $R_4$ représente H, Cl ou $OCH_3$.

14. Dérivé selon la revendication 13, caractérisé en ce qu'il s'agit du 7,8-dihydro-9-hydroxyimino-5-méthoxy-pyranno [2,3-g] indole.

15. Dérivé selon la revendication 1 et répondant à la formule $I_0$, caractérisé en ce que X représente $>CH-NH_2$, $R_2$ et $R_3$ représentent chacun l'atome d'hydrogène, $R_1$ représente l'atome d'hydrogène ou un groupe alkyle en $C_1$-$C_4$ et $R_4$ représente H, Cl ou $OCH_3$.

16. Dérivé selon la revendication 15, caractérisé en ce qu'il s'agit du 9-amino-5-méthoxy-7,8,9-trihydropyranno [2,3-g] indole.

17. Dérivé selon la revendication 3, caractérisé en ce qu'il s'agit du 5-chloro-1-(3-N,N-diméthylamino-propyl)-7,8,9-trihydropyranno [2,3-g] indole.

18. Composition thérapeutique caractérisée en ce qu'elle renferme, en association avec un excipient physiologiquement acceptable, au moins un dérivé de pyranno-indole ou l'un de ses sels d'addition d'acide selon la revendication 1.

19. Procédé de préparation d'un dérivé de pyranno-indole selon la revendication 1, caractérisé en ce que

— on fait réagir un aldéhyde de formule :

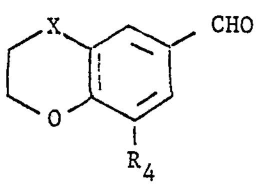

(VII)

où X et $R_4$ sont définis comme ci-dessus avec l'azidoacétate d'éthyle en présence d'un alcool inférieur en $C_1$-$C_2$ et d'un métal alcalin choisi parmi Na et K, à une température de − 5 °C et + 5 °C, pendant au moins 3 heures, pour obtenir un azide de formule :

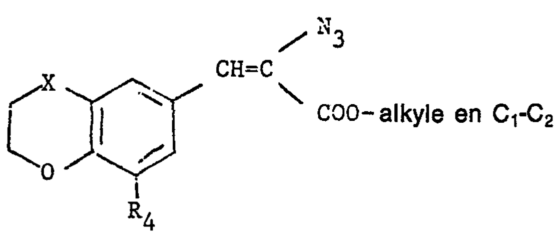

(VIII)

— on cyclise ledit azide en présence d'un solvant inerte choisi parmi le benzène, le toluène et le xylène, à la température de reflux du milieu réactionnel pendant au moins 1 heure, pour obtenir un dérivé pyranno-indolecarboxylate de formule :

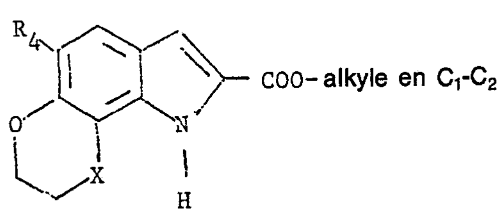

(IX)

— on saponifie l'ester de formule IX pour obtenir l'acide correspondant de formule :

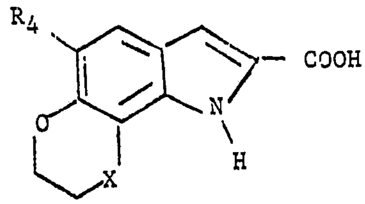

(X)

— on décarboxyle ledit acide au moyen d'un catalyseur, le chromite de cuivre, en présence d'un solvant inerte pour obtenir (i) un mélange de composés de formule

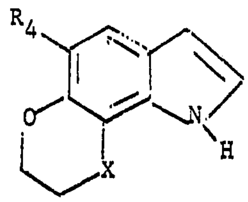

(XI)

et

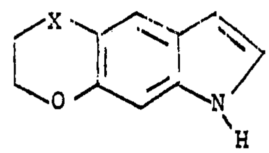

(XI')

quand $R_4 = H$ que l'on sépare par chromatographie, et (ii) uniquement le composé XI lorsque $R_4$ est différent de H,

— puis, si nécessaire, on introduit au moins un des groupes $R_1$ et $R_3$ différents de H pour obtenir à partir de XI et XI' respectivement :

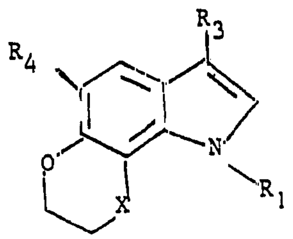

(XII)

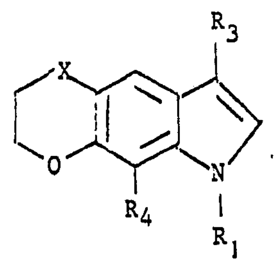

(XII')

et, le cas échéant, on introduit le groupe $R_4$ différent de H pour obtenir le composé XII' à partir du composé XI', et

— si nécessaire on transforme la fonction $R_2$ = carboxylate en fonction ester et amide par transestérification et transamidification, on transforme la fonction $R_2$ = COOH en fonction ester et amide par estérification et amidification, et transforme ladite fonction $R_2$ = COOH en $R_2$ = H par décarboxylation.

**Revendications** (pour l'Etat contractant AT)

1. Procédé de préparation d'un nouveau dérivé appartenant à la famille des pyranno-indoles, utile en thérapeutique et choisi parmi l'ensemble constitué par

(i) les pyranno [2,3-g] indoles de formule générale :

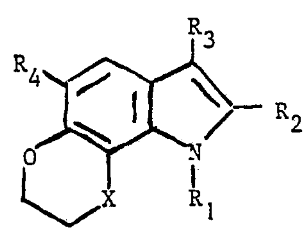

$(I_0)$

dans laquelle

X représente un groupe >CH₂, >CHOH, >CO,

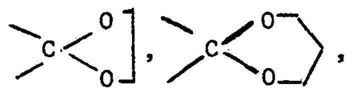

$>$C = NR (où R est un groupe OH ou alkoxy en $C_1$-$C_4$) ou $>$CH—NR'R'' (où R' et R'', identiques ou différents, représentent chacun H ou un alkyle en $C_1$-$C_4$) ;

$R_1$ représente l'atome d'hydrogène, un groupe alkyle en $C_1$-$C_4$ ou un groupe aminoalkylène $-(CH_2)_n-NR'R''$ dans lequel n est un nombre entier compris entre 1 et 4 et R' et R'' sont définis comme ci-dessus ;

$R_2$ représente l'atome d'hydrogène ou un groupe COY (où Y est OH ou alkoxy en $C_1$-$C_4$) ;

$R_3$ représente l'atome d'hydrogène, un groupe CHO, $(CH_2)_nNR'R''$ ou CO—CONR'R'' (où n, R' et R'' sont définis comme ci-dessus), et

$R_4$ représente l'atome d'hydrogène, un atome d'halogène, un groupe alkyle en $C_1$-$C_4$ ou alkoxy en $C_1$-$C_4$ ;

(ii) les pyranno [3,2-f] indoles de formule générale

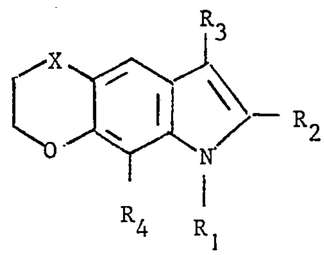

$(I_0')$

où X, $R_1$, $R_2$, $R_3$ et $R_4$ sont définis comme ci-dessus ;
et

(iii) leurs sels d'addition d'acide ;

ledit procédé étant caractérisé en ce que

— on fait réagir un aldéhyde de formule :

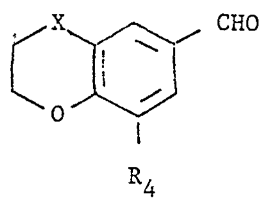

(VII)

où X et $R_4$ sont définis comme ci-dessus avec l'azidoacétate d'éthyle en présence d'un alcool inférieur en $C_1$-$C_2$ et d'un métal alcalin choisi parmi Na et K, à une température de − 5 °C et + 5 °C, pendant au moins 3 heures, pour obtenir un azide de formule :

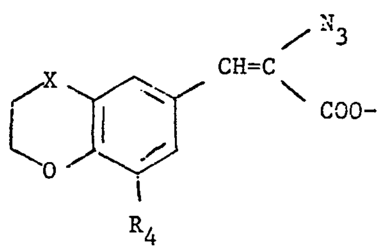

COO-alkyle en $C_1$-$C_2$ (VIII)

— on cyclise ledit azide en présence d'un solvant inerte choisi parmi le benzène, le toluène et le xylène, à la température de reflux du milieu réactionnel pendant au moins 1 heure, pour obtenir un dérivé pyranno-indolecarboxylate de formule :

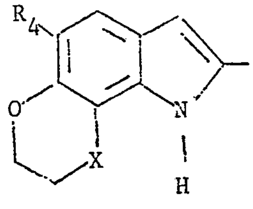

COO-alkyle en $C_1$-$C_2$ (IX)

— on saponifie l'ester de formule IX pour obtenir l'acide correspondant de formule :

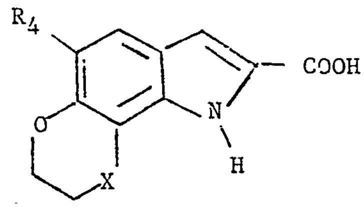

(X)

— on décarboxyle ledit acide au moyen d'un catalyseur, le chromite de cuivre, en présence d'un solvant inerte pour obtenir (i) un mélange de composés de formule

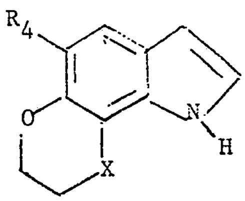

et

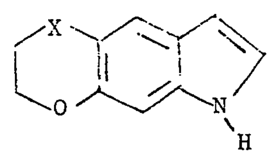

,

(XI) (XI')

quand $R_4 = H$ que l'on sépare par chromatographie, et (ii) uniquement le composé XI lorsque $R_4$ est différent de H,

— puis, si nécessaire, on introduit au moins un des groupes $R_1$ et $R_3$ différents de H pour obtenir à partir de XI et XI' respectivement :

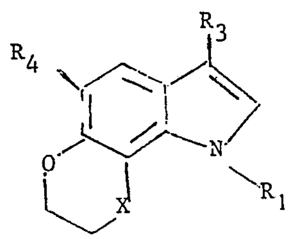

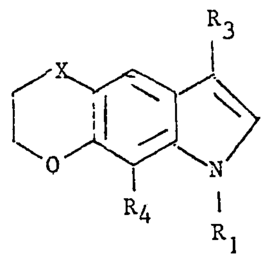

(XII) (XII')

et, le cas échéant, on introduit le groupe $R_4$ différent de H pour obtenir le composé XII' à partir du composé XI', et

— si nécessaire on transforme la fonction $R_2$ = carboxylate en fonction ester et amide par transestérification et transamidification, on transforme la fonction $R_2$ = COOH en fonction ester et amide par estérification et amidification, et transforme ladite fonction $R_2$ = COOH en $R_2$ = H par décarboxylation.

2. Procédé selon la revendication 1, caractérisé en ce que X est $>CH_2$, $>CO$,

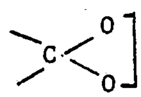

,

$>C = NOH$, $>CHOH$ ou $>CHNH_2$ ; $R_2$ est H, COOH, CO-alkoxy en $C_1$-$C_4$ ; $R_3$ est H, CHO, $CH_2N(CH_3)_2$ ou CO—$CON(CH_3)_2$ ; $R_4$ est H, Cl, Br, alkyle en $C_1$-$C_4$ ou alkoxy en $C_1$-$C_4$ ; et $R_1$ est H, alkyle en $C_1$-$C_4$ ou $(CH_2)_nNR'R''$ (où n, R' et R'' sont définis comme ci-dessus).

3. Procédé selon la revendication 1 pour la préparation d'un dérivé de formule $I_0$, caractérisé en ce que X représente $>CH_2$, $R_2$ représente H, COOH ou $COOCH_3$, $R_3$ représente H, CHO, CO—$CON(CH_3)_2$ ou $CH_2N(CH_3)_2$, $R_4$ représente H, Cl ou $OCH_3$, et $R_1$ représente H, $CH_3$ ou un groupe $(CH_2)_nNR'R''$ (où n, R' et R'' sont définis comme indiqué ci-dessus).

4. Procédé selon la revendication 1 pour la préparation d'un dérivé de formule $I_0$, caractérisé en ce que X représente $>CO$, $R_2$ représente H, COOH ou $COOCH_3$, $R_3$ représente H, CHO ou $CH_2N(CH_3)_2$, $R_4$ représente H, Cl ou $OCH_3$ et $R_1$ représente l'atome d'hydrogène.

5. Procédé selon la revendication 1 pour la préparation d'un dérivé de formule $I_0$, caractérisé en ce que X représente

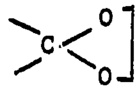,

$R_2$ représente $COOCH_3$, $R_4$ représente H, Cl ou $OCH_3$, et $R_1$ et $R_3$ représentent chacun l'atome d'hydrogène.

6. Procédé selon la revendication 1 pour la préparation d'un dérivé de formule $I'_0$, caractérisé en ce que X est $CH_2$, $R_3$ est H ou CHO, $R_1$ et $R_2$ représentent chacun l'atome d'hydrogène, et $R_4$ représente l'atome d'hydrogène ou de chlore.

7. Procédé selon la revendication 1 pour préparer un dérivé de formule $I_0$, caractérisé en ce que X représente $\gtrdot C = NR$ (où R est un groupe OH ou alkoxy en $C_1$-$C_4$), $R_2$ et $R_3$ représentent chacun l'atome d'hydrogène, $R_1$ représente l'atome d'hydrogène ou un groupe alkyle en $C_1$-$C_4$ et $R_4$ représente H, Cl ou $OCH_3$.

8. Procédé selon la revendication 1 pour préparer un dérivé de formule $I_0$, caractérisé en ce que X représente $\gtrdot CH-NH_2$, $R_2$ et $R_3$ représentent chacun l'atome d'hydrogène, $R_1$ représente l'atome d'hydrogène ou un groupe alkyle en $C_1$-$C_4$ et $R_4$ représente H, Cl ou $OCH_3$.

**Claims** (for the Contracting States : BE, CH, DE, FR, GB, IT, LI, LU, NL SE)

1. Derivative belonging to the family of pyranno-indoles and particularly useful in therapeutics, characterized in that it is selected from the group constituted by :

(i) [2,3-g] pyranno-indoles of general formula

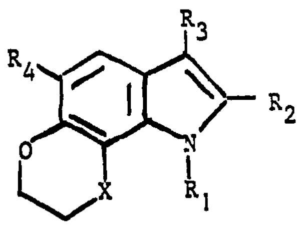

($I_0$)

wherein

X is a $\gtrdot CH_2$, $\gtrdot CHOH$, $\gtrdot CO$,

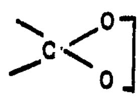, 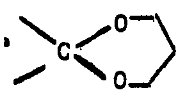,

$\gtrdot C = NR$ (wherein R is an OH or alkoxy group in $C_1$-$C_4$) or $\gtrdot CH-NR'R''$ (wherein R' and R'', identical or different, are each H or an alkyl in $C_1$-$C_4$) ;

$R_1$ is the hydrogen atom, an alkyl group in $C_1$-$C_4$ or an amino-alkylene group $-(CH_2)_n-NR'R''$ wherein n is an integer between 1 and 4 and R' and R'' are as defined above ;

$R_2$ is the hydrogen atom or a COY group (wherein Y is OH or alkoxy in $C_1$-$C_4$) ;

$R_3$ is the hydrogen atom, a CHO group, $(CH_2)_nNR'R''$ or $CO-CONR'R''$ (wherein n, R' and R'' are as defined above), and

$R_4$ is the hydrogen atom, a halogen atom, an alkyl group in $C_1$-$C_4$ or alkoxy in $C_1$-$C_4$ ;

(ii) the [3,2-f] pyranno-indoles of general formula :

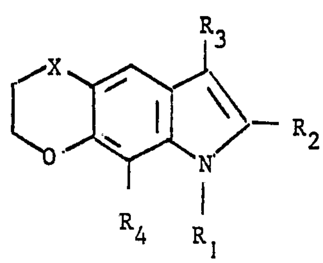

($I'_0$)

wherein, X, $R_1$, $R_2$, $R_3$ and $R_4$ are as defined above ;

and (iii) their acid addition salts.

2. Derivative according to claim 1, characterized in that X is $\gtrdot CH_2$, $\gtrdot CO$,

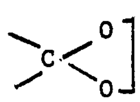,

>C = NOH, >CHOH or >CHNH$_2$ ; $R_2$ is H, COOH, CO-alkoxy in $C_1$-$C_4$ ; $R_3$ is H, CHO, $CH_2N(CH_3)_2$ or CO—$CON(CH_3)_2$ ; $R_4$ is H, Cl, Br, alkyl in $C_1$-$C_4$ or alkoxy in $C_1$-$C_4$ ; $R_1$ is H, alkyl in $C_1$-$C_4$ or $(CH_2)_nNR'R''$ (wherein n, R' and R'' are as defined above).

3. Derivative according to claim 1, and of formula $I_0$, characterized in that X is >$CH_2$, $R_2$ is H, COOH or $COOCH_3$, $R_3$ is H, CHO, CO—$CON(CH_3)_2$ or $CH_2N(CH_3)_2$, $R_4$ is H, Cl or $OCH_3$, and $R_1$ is H, $CH_3$ or a group $(CH_2)_nNR'R''$ (wherein n, R' and R'' are as defined above).

4. Derivative according to claim 1, and of formula $I_0$, characterized in that X is >CO, $R_2$ is H, COOH or $COOCH_3$, $R_3$ is H, CHO or $CH_2N(CH_3)_2$, $R_4$ is H, Cl or $OCH_3$, and $R_1$ is the hydrogen atom.

5. Derivative according to claim 1, and of formula $I_0$, characterized in that X is

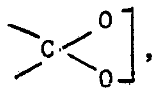,

$R_2$ is $COOCH_3$, $R_4$ is H, Cl or $OCH_3$, and $R_1$ and $R_3$ each are the hydrogen atom.

6. Derivative according to claim 1, and of formula $I'_0$, characterized in that X is $CH_2$, $R_3$ is H or CHO, $R_1$ and $R_2$ each are the hydrogen atom, and $R_4$ is the hydrogen or chlorine atom.

7. Derivative according to claim 3, characterized in that it is 5-chloro-7,8,9-trihydropyranno [2,3-g] indole-2-carboxylic acid.

8. Derivative according to claim 3, characterized in that it is 5-methoxy-7,8,9-trihydropyranno [2,3-g] indole.

9. Derivative according to claim 3, characterized in that it is 3-(7,8,9-trihydropyranno [2,3-g] indolyl)-N,N-dimethyl-methylamine and its acid addition salts.

10. Derivative according to claim 4, characterized in that it is 5-methoxy-9-oxo-7,8-dihydropyranno [2,3-g] indole.

11. Derivative according to claim 4, characterized in that it is 5-methoxy-9-oxo-7,8-dihydro-pyranno [2,3-g] indole-2-carboxylic acid.

12. Derivative according to claim 6, characterized in that it is 5,6,7-trihydropyranno [3,2-f] indole-3-carboxaldehyde.

13. Derivative according to claim 1, and of formula $I_0$, characterized in that X is >C = NR (wherein R is an OH oralkoxy in $C_1$-$C_4$ group), $R_2$ and $R_3$ each are the hydrogen atom, $R_1$ is the hydrogen atom or an alkyl group in $C_1$-$C_4$ and $R_4$ is H, Cl or $OCH_3$.

14. Derivative according to claim 13, characterized in that it is 7,8-dihydro-9-hydroxyimino-5-methoxy-pyranno [2,3-g] indole.

15. Derivative according to claim 1, and of formula $I_0$, characterized in that X is >CH—$NH_2$, $R_2$ and $R_3$ are each the hydrogen atom, $R_1$ is the hydrogen atom or an alkyl group in $C_1$-$C_4$ and $R_4$ is H, Cl or $OCH_3$.

16. Derivative according to claim 15, characterized in that it is 9-amino-5-methoxy-7,8,9-trihydropyranno [2,3-g] indole.

17. Derivative according to claim 3, characterized in that it is 5-chloro-1-(3,N,N-dimethylaminopropyl)-7,8,9-trihydropyranno [2,3-g] indole.

18. Therapeutical composition characterized in that it contains, in association with a physiologically acceptable excipient, at least one derivative of pyrannoindole or one of its acid addition salts according to claim 1.

19. Process for the preparation of a derivative of pyranno-indole according to claim 1, characterized in that :

— an aldehyde of formula :

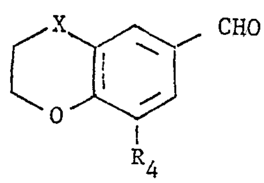

(VII)

wherein X and $R_4$ are as defined above, is reacted with ethyl azidoacetate in the presence of a lower alcohol in $C_1$-$C_2$ and an alkali metal selected from Na and K, at a temperature of − 5 °C and + 5 °C, for at least 3 hours, to obtain an azid of formula :

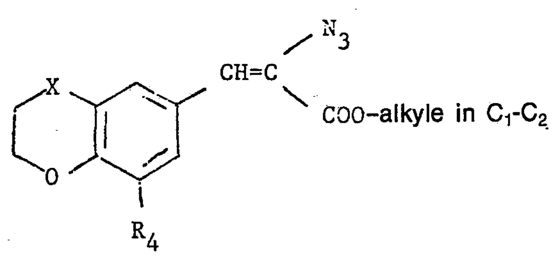

(VIII)

— said azid is cyclized in the presence of an inert solvent selected from benzene, toluene and xylene, at the reflux temperature of the reaction medium for at least one hour, to obtain a pyrrano-indolecarboxylate derivative of formula :

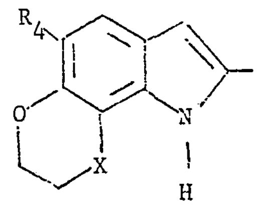

— COO-alkyle in $C_1$-$C_2$ (IX)

— the ester of formula IX is saponified to obtain the corresponding acid of formula :

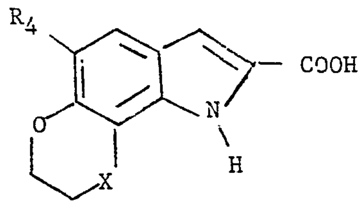

(X)

— said acid is decarboxylated by means of a catalyst, namely copper chromite, in the presence of an inert solvent to obtain (i) a mixture of compounds of formula :

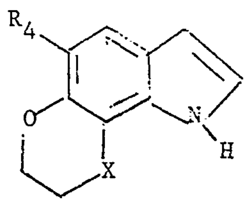

and

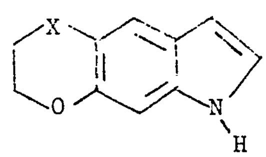

(XI) (XI')

when $R_4$ = H which is separated by chromatography, and (ii) the compound XI only when $R_4$ is different from H,

— then, if necessary, at least one of the groups $R_1$ and $R_3$ different from H is introduced, to obtain from XI and XI' respectively :

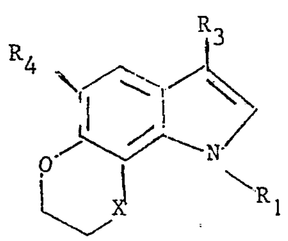

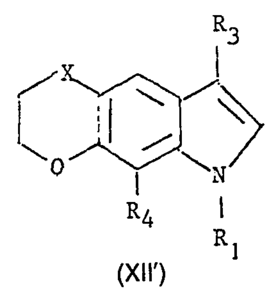

(XII) (XII')

and, optionally, the group $R_4$ different from H is introduced to obtain compound XII' from compound XI', and

— if necessary, function $R_2$ = carboxylate is transformed into an ester and amide function by transesterification and transamidation, function $R_2$ = COOH is transformed into an ester and amide function by esterification and amidation, and said function $R_2$ = COOH is transformed into $R_2$ = H by decarboxylation.

**Claims** (for the Contracting State AT)

1. Preparation process of a new derivative belonging to the family of pyranno-indols, useful in therapeutics and selected from the group constituted by :

(i) [2,3-g] pyranno-indols of general formula

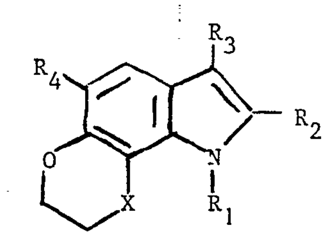

($I_0$)

wherein

X is a $\geqq CH_2$, $\geqq CHOH$, $\geqq CO$,

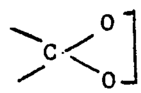, 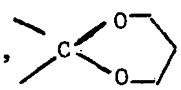,

group,

$\geqq C = NR$ (wherein R is an OH or alkoxy group in $C_1$-$C_4$) $\geqq CH—NR'R''$ (wherein R' and R'', either identical or different are each H or an alkyl in $C_1$-$C_4$) ;

$R_1$ is the hydrogen atom, an alkyl group in $C_1$-$C_4$ or an aminoalkylene group $(CH_2)_n—NR'R''$ wherein n is an integer between 1 and 4 and R' and R'' are as defined above ;

$R_2$ is the hydrogen atom or a COY group (wherein Y is OH or alkoxy in $C_1$-$C_4$),

$R_3$ is the hydrogen atom, a CHO, $(CH_2)_nNR'R''$ or CO—CONR'R'' group (wherein n, R' and R'' are defined as above), and

$R_4$ is the hydrogen atom, a halogen atom, an alkyl group in $C_1$-$C_4$ or alkoxy group in $C_1$-$C_4$ ;

(ii) the [3,2-f] pyranno-indols of general formula

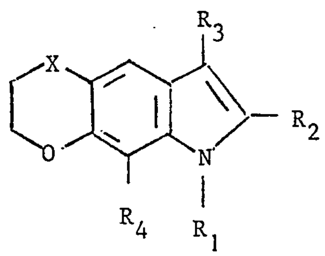

($I'_0$)

wherein X, $R_1$, $R_2$, $R_3$ and $R_4$ are as defined above ; and

(iii) their acid addition salts ;

said process being characterized in that

— an aldehyde of formula :

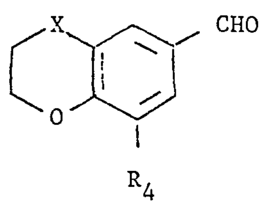

(VII)

wherein X and $R_4$ are as defined above, is reacted with the ethyl azidoacetate in the presence of a lower alcohol in $C_1$-$C_2$ and an alkali metal selected from Na and K at a temperature of − 5 °C and + 5 °C for at least 3 hours, to obtain an azide of formula

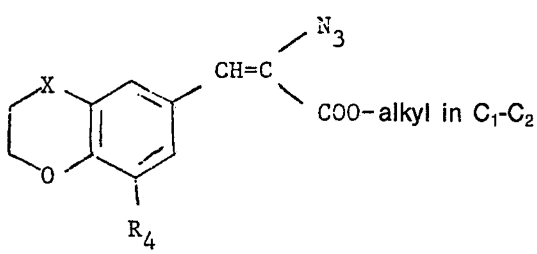

(VIII)

— said azide is cyclized in the presence of an inert solvent selected from benzene, toluene and xylene, at the reflux temperature of the reaction medium, for at least 1 hour, to obtain an pyranno-indolecarboxylate derivative of formula :

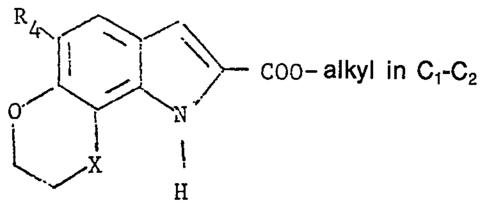

(IX)

— the ester of formula IX is saponified to obtain the corresponding acid of formula :

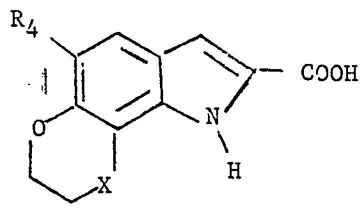

(X)

— said acid is decarboxylated by means of a catalyst, namely copper chromite, in the presence of an inert solvent to obtain (i) a mixture of compounds formulae :

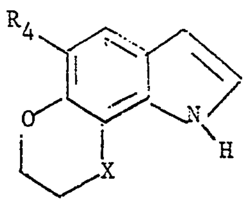

and

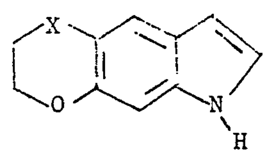

(XI) (XI′)

when $R_4 = H$ which is separated by chromatography, and (ii) compound XI only when $R_4$ is different from H,

— then, if necessary, at least one of the groups $R_1$ and $R_3$ different from H is introduced, to obtain from XI and XI′ respectively :

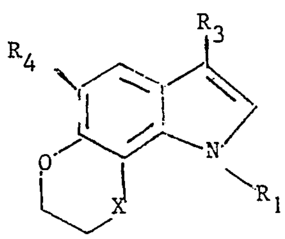

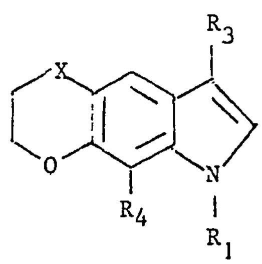

(XII) (XII′)

and, optionally the group $R_4$ which is different from H is introduced to obtain compound XII′ from compound XI′, and

— if necessary function $R_2$ = carboxylate is transformed into an ester and amide function by transesterification and transamidation, function $R_2$ = COOH is turned into an ester and amide function by esterification and amidation, and said function $R_2$ = COOH is transformed into $R_2$ = H by decarboxylation.

2. Process according to claim 1, characterized in that X is $>CH_2$, $>CO$,

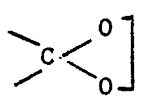,

$>C=NOH$, $>CHOH$ or $>CHNH_2$ ; $R_2$ is H, COOH, CO-alkoxy in $C_1$-$C_4$ ; $R_3$ is H, CHO, $CH_2N(CH_3)_2$ or $CO{-}CON(CH_3)_2$ ; $R_4$ is H, Cl, Br, alkyl in $C_1$-$C_4$, or alkoxy in $C_1$-$C_4$ ; and $R_1$ is H, alkyl in $C_1$-$C_4$ or $(CH_2)_n$ NR′R″ (wherein n, R′ and R″ are as defined above).

3. Process according to claim 1, for the preparation of a derivative of formula $I_0$, characterized in that X is $\geq CH_2$, $R_2$ is H, COOH or $COOCH_3$, $R_3$ is H, CHO, CO—$CON(CH_3)_2$ or $CH_2N(CH_3)_2$, $R_4$ is H, Cl or $OCH_3$, and $R_1$ is H, $CH_3$ or a group $(CH_2)_n$ NR'R" (wherein n, R' and R" are as defined above).

4. Process according to claim 1, for the preparation of a derivative of formula $I_0$, characterized in that X is $\geq CO$, $R_2$ is H, COOH or $COOCH_3$, $R_3$ is H, CHO or $CH_2N(CH_3)_2$, $R_4$ is H, Cl ou $OCH_3$ and $R_1$ is the hydrogen atom.

5. Process according to claim 1, for the preparation of a derivative of formula $I_0$, characterized in that X is

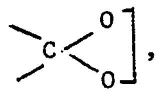

$R_2$ is $COOCH_3$, $R_4$ is H, Cl or $OCH_3$ and $R_1$ and $R_3$ each are the hydrogen atom.

6. Process according to claim 1, for the preparation of a derivative of formula $I'_0$, characterized in that X is $CH_2$, $R_3$ is H or CHO, $R_1$ and $R_2$ each are the hydrogen atom, and $R_4$ is the chloride or hydrogen atom.

7. Process according to claim 1 for preparing a derivative of formula $I_0$, characterized in that X is $\geq C = NR$ (wherein R is an OH or alkoxy group in $C_1$-$C_4$), $R_2$ and $R_3$ each are the hydrogen atom, $R_1$ is the hydrogen atom or an alkyl in $C_1$-$C_4$ and $R_4$ is H, Cl or $OCH_3$.

8. Process according to claim 1 for preparing a derivative of formula $I_0$, characterized in that X is $\geq CH—NH_2$, $R_2$ and $R_3$ are each the hydrogen atom, $R_1$ is the hydrogen atom or an alkyl group in $C_1$-$C_4$ and $R_4$ is H, Cl or $OCH_3$.

**Ansprüche** (für die Vertragsstaaten : BE, CH, DE, FR, GB, IT, LI, LU, NL, SE)

1. Zur Familie der Pyranoindole gehörendes und insbesondere in der Therapie wertvolles Derivat, dadurch gekennzeichnet, daß es ausgewählt ist aus der Gruppe bestehend aus

(i) Pyrano [2,3-g] indole der allgemeinen Formel

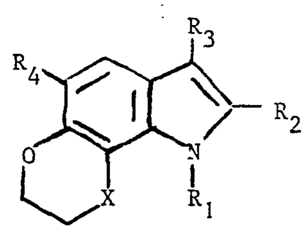

($I_0$)

worin

X eine Gruppe $\geq CH_2$, $\geq CHOH$, $\geq CO$,

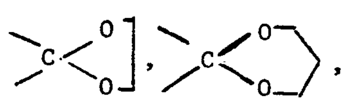

$\geq C = NR$ (worin R eine OH- oder eine $C_1$-$C_4$-Alkoxygruppe ist) oder $\geq CH—NR'R''$ (worin R' und R", gleich oder verschieden, jeweils H oder $C_1$-$C_4$-Alkyl bedeuten) darstellt ;

$R_1$ für Wasserstoffatom, eine $C_1$-$C_4$-Alkylgruppe oder eine $—(CH_2)_n—NR'R''$-Aminoalkylengruppe, worin n eine ganze Zahl zwischen 1 und 4 bedeutet und R' und R" obige Bedeutung haben, steht ;

$R_2$ Wasserstoffatom oder eine COY-Gruppe (worin Y OH oder $C_1$-$C_4$-Alkoxy ist) darstellt ;

$R_3$ Wasserstoffatom, eine Gruppe CHO, $(CH_2)_nNR'R''$ oder CO-CONR'R" (worin n, R' und R" obige Bedeutung haben) darstellt und

$R_4$ Wasserstoffatom, ein Halogenatom, eine $C_1$-$C_4$-Alkyl- oder $C_1$-$C_4$-Alkoxygruppe bedeutet ;

(ii) Pyrano [3,2-f] indole der allgemeinen Formel

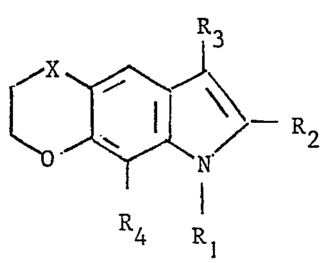

($I'_0$)

worin X, $R_1$, $R_2$, $R_3$ und $R_4$ obige Bedeutung haben ; und

(iii) ihren Säureadditionssalzen.

2. Derivat nach Anspruch 1, dadurch gekennzeichnet, daß X $>CH_2$, $>CO$,

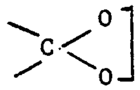,

$>C = NOH$, $>CHOH$ oder $>CHNH_2$ ist ; $R_2$ H, COOH, CO-$C_1$-$C_4$-Alkoxy ist ; $R_3$ H, CHO, $CH_2N(CH_3)_2$ oder CO—$CON(CH_3)_2$ ist ; $R_4$ für H, Cl, Br, $C_1$-$C_4$-Alkyl oder $C_1$-$C_4$-Alkoxy steht und $R_1$ H, $C_1$-$C_4$-Alkyl oder $(CH_2)_nNR'R''$ (worin n, R' und R'' obige Bedeutung haben) darstellt.

3. Derivat nach Anspruch 1 gemäß der Formel $I_0$, dadurch gekennzeichnet, daß X $>CH_2$ bedeutet, $R_2$ H, COOH oder $COOCH_3$ darstellt, $R_3$ H, CHO, CO—$CON(CH_3)_2$ oder $CH_2H(CH_3)_2$ darstellt, $R_4$ für H, Cl oder $OCH_3$ steht und $R_1$ H, $CH_3$ oder eine $(CH_2)_nNR'R''$-Gruppe (worin n, R' und R'' obige Bedeutung haben) darstellt.

4. Derivat nach Anspruch 1 und gemäß der Formel $I_0$, dadurch gekennzeichnet, daß X $>CO$ ist, $R_2$ H, COOH oder $COOCH_3$ bedeutet, $R_3$ für H, CHO oder $CH_2N(CH_3)_2$ steht, $R_4$ H, Cl oder $OCH_3$ darstellt und $R_1$ Wasserstoffatom bedeutet.

5. Derivat nach Anspruch 1 und gemäß der Formel $I_0$, dadurch gekennzeichnet, daß X

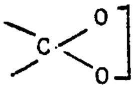,

darstellt, $R_2$ $COOCH_3$ bedeutet, $R_4$ für H, Cl oder $OCH_3$ steht und $R_1$ und $R_3$ jeweils Wasserstoffatom bedeuten.

6. Derivat nach Anspruch 1 und gemäß der Formel $I'_0$, dadurch gekennzeichnet, daß X $CH_2$ ist, $R_3$ H oder CHO ist, $R_1$ und $R_2$ jeweils Wasserstoffatom darstellen und $R_4$ Wasserstoff- oder Chloratom bedeutet.

7. Derivat nach Anspruch 3, dadurch gekennzeichnet, daß es sich um 5-Chlor-7,8,9-trihydropyrano [2,3-g] indol-2-carbonsäure handelt.

8. Derivat nach Anspruch 3, dadurch gekennzeichnet, daß es sich um 5-Methoxy-7,8,9-trihydropyrano [2,3-g] indol handelt.

9. Derivat nach Anspruch 3, dadurch gekennzeichnet, daß es sich um 3-(7,8,9-Trihydropyrano [2,3-g] indolyl)-N,N-dimethylmethylamin und seine Säureadditionssalze handelt.

10. Derivat nach Anspruch 4, dadurch gekennzeichnet, daß es sich um 5-Methoxy-9-oxo-7,8-dihydropyrano [2,3-g]-indol handelt.

11. Derivat nach Anspruch 4, dadurch gekennzeichnet, daß es sich um 5-Methoxy-9-oxo-7,8-dihydropyrano [2,3-g]-indol-2-carbonsäure handelt.

12. Derivat nach Anspruch 6, dadurch gekennzeichnet, daß es sich um 5,6,7-Trihydropyrano [3,2-f] indol-3-carboxaldehyd handelt.

13. Derivat nach Anspruch 1 und gemäß der Formel $I_0$, dadurch gekennzeichnet, daß X $>C = NR$ (worin R eine OH- oder $C_1$-$C_4$-Alkoxygruppe ist) bedeutet, $R_2$ und $R_3$ jeweils Wasserstoffatom bedeuten, $R_1$ ein Wasserstoffatom oder eine $C_1$-$C_4$-Alkylgruppe darstellt und $R_4$ für H, Cl oder $OCH_3$ steht.

14. Derivat nach Anspruch 13, dadurch gekennzeichnet, daß es sich um 7,8-Dihydro-9-hydroxyimino-5-methoxy-pyrano [2,3-g] indol handelt.

15. Derivat nach Anspruch 1 und gemäß der Formel $I_0$, dadurch gekennzeichnet, daß X $>CH—NH_2$ bedeutet, $R_2$ und $R_3$ jeweils Wasserstoffatom bedeuten, $R_1$ Wasserstoffatom oder eine $C_1$-$C_4$-Alkylgruppe darstellt und $R_4$ für H, Cl oder $OCH_3$ steht.

16. Derivat nach Anspruch 15, dadurch gekennzeichnet, daß es sich um 9-Amino-5-methoxy-7,8,9-trihydropyrano-[2,3-g] indol handelt.

17. Derivat nach Anspruch 3, dadurch gekennzeichnet, daß es sich um 5-Chlor-1-(3-N,N-dimethylaminopropyl)-7,8,9-trihydropyrano [2,3-g] indol handelt.

18. Therapeutische Zusammensetzung, dadurch gekennzeichnet, daß sie in Verbindung mit einem physiologisch akzeptablen Träger zumindest ein Pyranoindolderivat oder eines seiner Säureadditionssalze gemäß Anspruch 1 enthält.

19. Verfahren zur Herstellung eines Pyranoindolderivats nach Anspruch 1, dadurch gekennzeichnet, daß

— man ein Aldehyd der Formel

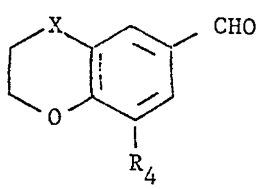

(VII)

worin X und $R_4$ obige Bedeutung haben, mit dem Äthylazidoacetat in Gegenwart eines $C_1$-$C_2$-Niedrigalkohols und eines Alkalimetalls, ausgewählt aus Na und K, bei einer Temperatur zwischen – 5 °C und + 5 °C während wenigstens 3 h zur Umsetzung bringt, um ein Azid der Formel

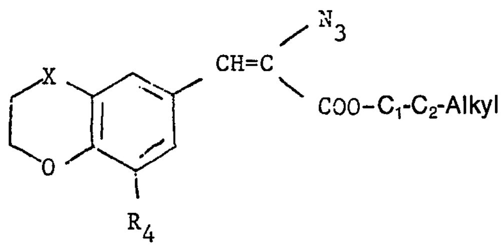

(VIII)

zu erhalten,

— man das Azid in Gegenwart eines inerten Lösungsmittels, ausgewählt aus Benzol, Toluol und Xylol, bei der Rückflußtemperatur des Reaktionsmediums während zumindest 1 h cyclisiert, um ein Pyranoindolcarboxylatderivat der Formel

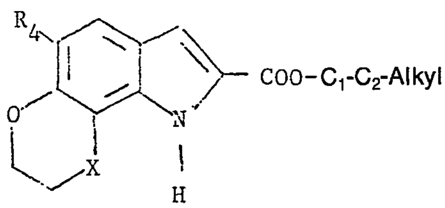

(IX)

zu erhalten,

— man den Ester der Formel IX zur Gewinnung der entsprechenden Säure der Formel

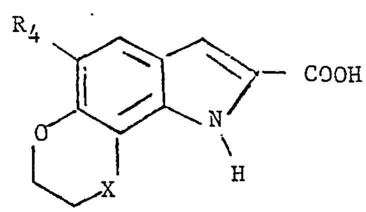

(X)

verseift,

— man die Säure mittels eines Katalysators, Kupferchromit, in Gegenwart eines inerten Lösungsmittels decarboxyliert zur Gewinnung (i) einer Mischung von Verbindungen der Formel

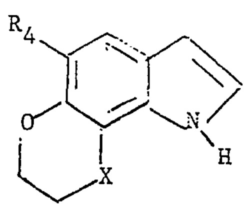

und

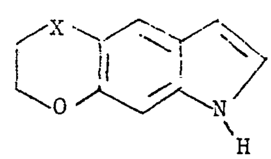

(XI) (XI')

wenn $R_4$ = H, welche durch Chromatografie getrennt wird, und (ii) ausschließlich der Verbindung XI, wenn $R_4$ nicht H ist,

— man danach gegebenenfalls zumindest eine der von H verschiedenen Gruppen $R_1$ und $R_3$ einführt, um aus XI bzw. XI'

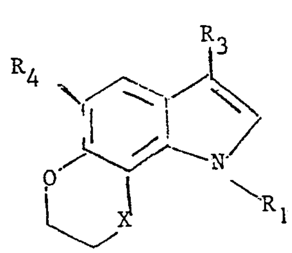

und

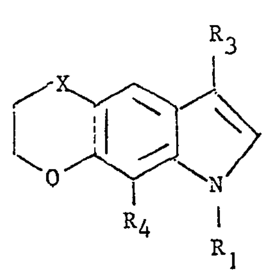

(XII) (XII')

zu gewinnen, und man gegebenenfalls die von H verschiedene Gruppe $R_4$ einführt, um aus der Verbindung XI' die Verbindung XII' zu gewinnen, und

— man gegebenenfalls die Funktion $R_2$ = Carboxylat durch Umesterung und Umamidierung in die Ester- und Amidfunktion überführt, die Funktion $R_2$ = COOH durch Veresterung und Amidierung in die Ester- und Amidfunktion überführt und die Funktion $R_2$ = COOH durch Decarboxylierung in $R_2$ = H überführt.

**Ansprüche** (für den Vertragsstaat AT)

1. Verfahren zur Herstellung eines neuen, zur Familie der Pyranoindole gehörenoen Derivats, das in der Therapie wertvoll und ausgewählt ist aus der Gruppe

i) Pyrano [2,3-g] indole der allgemeinen Formel

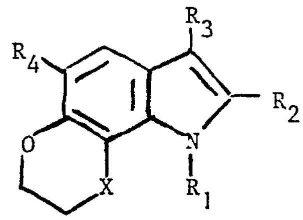

($I_0$)

worin

X eine Gruppe $>CH_2$, $>CHOH$, $>CO$,

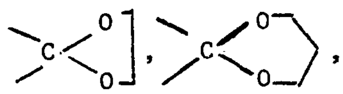

$>C=NR$ (worin R eine OH- oder eine $C_1$-$C_4$-Alkoxygruppe ist) oder $>CH—NR'R''$ (worin R' und R'', gleich oder verschieden, jeweils H oder $C_1$-$C_4$-Alkyl bedeuten) darstellt ;

$R_1$ für Wasserstoffatom, eine $C_1$-$C_4$-Alkylgruppe oder eine $—(CH_2)_n—NR'R''$-Aminoalkylengruppe, worin n eine ganze Zahl zwischen 1 und 4 bedeutet und R' und R'' obige Bedeutung haben, steht ;

$R_2$ Wasserstoffatom oder eine COY-Gruppe (worin Y OH oder $C_1$-$C_4$-Alkoxy ist) darstellt ;

$R_3$ Wasserstoffatom, eine Gruppe CHO, $(CH_2)_nNR'R''$ oder CO—CONR'R'' (worin n, R' und R'' obige Bedeutung haben) darstellt und

$R_4$ Wasserstoffatom, ein Halogenatom, eine $C_1$-$C_4$-Alkyl- oder $C_1$-$C_4$-Alkoxygruppe bedeutet ;

ii) Pyrano [3,2-f] indole der allgemeinen Formel

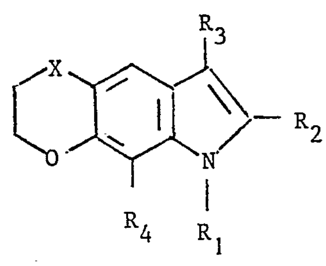

($I'_0$)

worin X, $R_1$, $R_2$, $R_3$ und $R_4$ obige Bedeutung haben ; und

iii) ihren Säureadditionssalzen ;

welches Verfahren dadurch gekennzeichnet ist, daß

— man ein Aldehyd der Formel

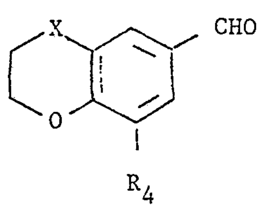

(VII)

worin X und $R_4$ obige Bedeutung haben, mit dem Äthylazidoacetat in Gegenwart eines $C_1$-$C_2$-Niedrigalkohols und eines Alkalimetalls, ausgewählt aus Na und K, bei einer Temperatur zwischen − 5 °C und + 5 °C während wenigstens 3 h zur Umsetzung bringt, um ein Azid der Formel

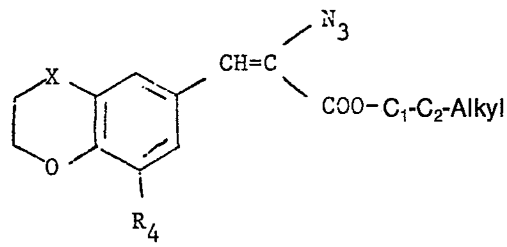

(VIII)

zu erhalten,

— man das Azid in Gegenwart eines inerten Lösungsmittels, ausgewählt aus Benzol, Toluol und Xylol, bei der Rückflußtemperatur des Reaktionsmediums während zumindest 1 h cyclisiert, um ein Pyranoindolcarboxylatderivat der Formel

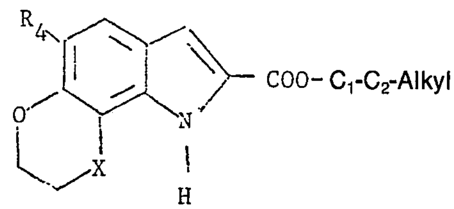

(IX)

zu erhalten,

— man den Ester der Formel IX zur Gewinnung der entsprechenden Säure der Formel

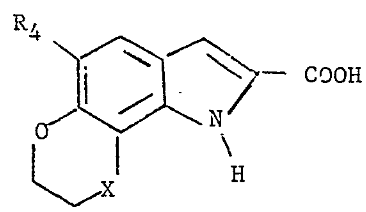

(X)

verseift,

— man die Säure mittels eines Katalysators, Kupferchromit, in Gegenwart eines inerten Lösungsmittels dacarboxyliert, zur Gewinnung (i) einer Mischung von Verbindungen der Formel

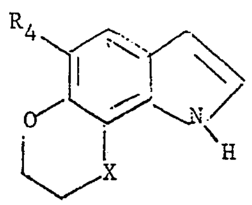

und

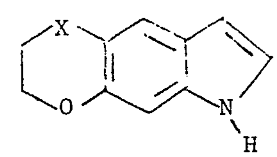

(XI) (XI')

wenn $R_4 = H$, welche durch Chromatografie getrennt wird, und (ii) ausschließlich der Verbindung XI, wenn $R_4$ von H verschieden ist,

— man danach gegebenenfalls zumindest eine der von H verschiedenen Gruppen $R_1$ und $R_3$ einführt, um aus XI bzw. XI'

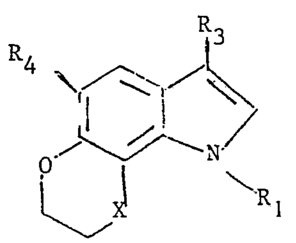

und

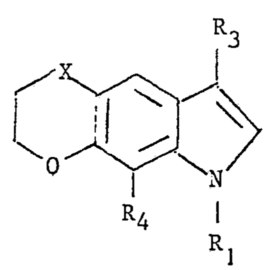

(XII) (XII')

zu gewinnen, und man gegebenenfalls die von H verschiedene Gruppe $R_4$ einführt, um aus der Verbindung XI' die Verbindung XII' zu gewinnen, und

— man gegebenenfalls die Funktion $R_2$ = Carboxylat durch Umesterung und Umamidierung in die Ester- und Amidfunktion überführt, die Funktion $R_2 = COOH$ durch Veresterung und Amidierung in die Ester- und Amidfunktion überführt und die Funktion $R_2 = COOH$ durch Decarboxylierung in $R_2 = H$ überführt.

2. Verfahren nach Anspruch 1, dadurch gekennzeichnet, daß $X{=}CH_2$, ${=}CO$,

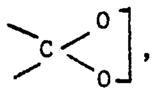,

${=}C = NOH$, ${=}CHOH$ oder ${=}CHNH_2$ ist ; $R_2$ H, COOH, CO—$C_1$-$C_4$-Alkoxy ist ; $R_3$ H, CHO, $CH_2N(CH_3)_2$ oder CO—$CON(CH_3)_2$ ist ; $R_4$ für H, Cl, Br, $C_1$-$C_4$-Alkyl oder $C_1$-$C_4$-Alkoxy steht und $R_1$ H, $C_1$-$C_4$-Alkyl oder $(CH_2)_nNR'R''$ (worin n, R' und R'' obige Bedeutung haben) darstellt.

3. Verfahren nach Anspruch 1 zur Herstellung eines Derivats der Formel $I_0$, dadurch gekennzeichnet, daß $X{=}CH_2$ bedeutet, $R_2$ H, COOH oder $COOCH_3$ darstellt, $R_3$ H, CHO, CO—$CON(CH_3)_2$ oder $CH_2N(CH_3)_2$ darstellt, $R_4$ für H, Cl oder $OCH_3$, steht und $R_1$ H, $CH_3$ oder eine $(CH_2)_nNR'R''$-Gruppe (worin n, R' und R'' obige Bedeutung haben) darstellt.

4. Verfahren nach Anspruch 1 zur Herstellung eines Derivats der Formel $I_0$, dadurch gekennzeichnet, daß $X{=}CO$ ist, $R_2$ H, COOH oder $COOCH_3$ bedeutet, $R_3$ für H, CHO oder $CH_2N(CH_3)_2$ steht, $R_4$ H, Cl oder $OCH_3$ darstellt und $R_1$ Wasserstoffatom bedeutet.

5. Verfahren nach Anspruch 1 zur Herstellung eines Derivats der Formel $I_0$, dadurch gekennzeichnet, daß X

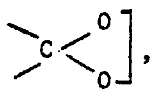,

darstellt, $R_2$ $COOCH_3$ bedeutet, $R_4$ für H, Cl oder $OCH_3$ steht und $R_1$ und $R_3$ jeweils Wasserstoffatom bedeuten.

6. Verfahren nach Anspruch 1 zur Herstellung eines Derivats der Formel $I'_0$, dadurch gekennzeichnet, daß X $CH_2$ ist, $R_3$ H oder CHO ist, $R_1$ und $R_2$ jeweils Wasserstoffatom darstellen und $R_4$ Wasserstoff- oder Chloratom bedeutet.

7. Verfahren nach Anspruch 1 zur Herstellung eines Derivats der Formel $I_0$, dadurch gekennzeichnet, daß $X{=}C = NR$ (worin R eine OH- oder $C_1$-$C_4$-Alkoxygruppe ist) bedeutet, $R_2$ und $R_3$ jeweils Wasserstoffatom bedeuten, $R_1$ ein Wasserstoffatom oder eine $C_1$-$C_4$-Alkylgruppe darstellt und $R_4$ für H, Cl oder $OCH_3$ steht.

8. Verfahren nach Anspruch 1 zur Herstellung eines Derivats der Formel $I_0$, dadurch gekennzeichnet, daß $X{=}CH—NH_2$ bedeutet, $R_2$ und $R_3$ jeweils Wasserstoffatom bedeuten, $R_1$ Wasserstoffatom oder eine $C_1$-$C_4$-Alkylgruppe darstellt und $R_4$ für H, Cl oder $OCH_3$ steht.